# FASCICULE DE BREVET EUROPEEN

(11) **EP 3 688 458 B1**
(45) Date de publication et mention de la délivrance du brevet: **25.01.2023**
(21) Numéro de dépôt: 18772840.7
(22) Date de dépôt: 26.09.2018
(51) Int. Cl.: G01N 33/18

(54) **DETECTION ET CARACTERISATION AMELIOREES D'ANOMALIES DANS UN CONTINUUM D'EAU**
VERBESSERTE DETEKTION UND CHARAKTERISIERUNG VON ANOMALIEN IN EINEM KONTINUUM VON WASSER
IMPROVED DETECTION AND CHARACTERIZATION OF ANOMALIES IN A CONTINUUM OF WATER

(30) Priorité: 29.09.2017 US 201762565722 P; 27.12.2017 FR 1763286
(43) Date de publication de la demande: 05.08.2020
(73) Titulaire: Suez International, 92040 Paris La Défense Cedex (FR)
(72) Inventeur: DO QUANG, Zdravka, Bailly 78870 (FR); CUSSONNEAU, Guillaume, 75009 Paris (FR); FAY, Gilles, 75019 Paris (FR)
(74) Mandataire: Atout PI Laplace
(86) Numéro de dépôt international: PCT/EP2018/076187
(87) Numéro de publication internationale: WO 2019/063648

(56) Documents cités:
- EP-A1- 3 112 960
- WO-A2-2004/063964
- US-A1- 2008 178 663

## Description

### DOMAINE DE L'INVENTION

La présente invention concerne le domaine de la gestion de l'eau, dans des réseaux de distribution d'eau ou des espaces naturels. Plus particulièrement, la présente invention concerne la détection d'anomalies affectant la qualité de l'eau.

### ETAT DE L'ART PRECEDENT

Les réseaux de distribution d'eaux peuvent être affectés par de nombreuses anomalies affectant la qualité de l'eau. Par exemple, l'eau peut être contaminée par différents polluants introduits volontairement ou involontairement par retour d'eau ou erreur de connexion, par des particules de matières se déposant régulièrement dans les conduites et soudainement remises en suspension, par la réaction avec les matériaux constitutifs des réseaux, ou par des bactéries lors de phénomènes de recroissances bactériennes. Afin de prévenir les effets délétères pouvant être causés par la contamination, ou de manière plus générale par la diminution de la qualité de l'eau pouvant provoquer l'inconfort des usagers (changement de couleur, de goût, d'odeur ...), il convient de détecter et caractériser, de manière aussi rapide et précise que possible, tout événement affectant la qualité de l'eau. Des problèmes altérant la qualité de l'eau peuvent également survenir dans des systèmes, étendues ou cours d'eau naturels tels que des lacs, étangs ou rivières ou ensemble d'étendues et de cours d'eaux naturels. Ces systèmes peuvent être affectés par exemple par des pollutions accidentelles, ou la croissance anormale d'algues, dégradant significativement leur état et empêchant leur usage (potabilisation, eaux de baignade ...).

La détection de telles anomalies peut dans certains cas s'avérer indispensable, afin d'engager des actions correctrices pour corriger l'anomalie. Les différentes anomalies survenant dans un système aquatique peuvent être détectées en surveillant un ensemble de paramètres physiques dans l'eau. Par exemple, une valeur anormalement basse des paramètres tels que la concentration en chlore ou anormalement haute pour la turbidité peut permettre de détecter une anomalie.

Cependant, cette tâche peut parfois s'avérer difficile, pour plusieurs raisons. D'une part, la détection d'une anomalie nécessite le déploiement de capteurs, pour disposer d'une information fine sur les paramètres physico-chimiques de l'eau. D'autre part, il peut être parfois difficile, à partir de mesures de capteurs, de distinguer des variations normales de paramètres physiques de l'eau, dues aux différentes opérations hydrauliques nécessaires au bon fonctionnement du réseau, de valeurs anormales traduisant une anomalie. En particulier, la configuration spécifique de chaque système peut lui conférer des valeurs spécifiques. Par exemple, la concentration en chlore peut différer significativement au sein d'un réseau de distribution d'eau potable, selon qu'on se situe proche d'une usine ou d'un point de chloration ou dans des zones éloignées ayant un long temps de séjour. La qualité de l'eau traitée peut également varier de manière plus ou moins régulière selon les différents types de ressources disponibles tout au long de l'année. Il s'agit alors de pouvoir détecter si un mélange inédit est rencontré.

Afin de proposer une détection plus fine des anomalies affectant un réseau de distribution d'eau, la demanderesse a déposé un brevet, publié sous le numéro WO 2016/012972. Le brevet WO 2016/012972 divulgue une méthode de détection d'anomalies dans un réseau de distribution particulièrement adaptée à un réseau de distribution d'eau potable, consistant à acquérir des mesures d'un ou plusieurs capteurs sur le réseau de distribution, former des séries temporelles de mesures capteurs, extraire des caractéristiques opérationnelles de mesures capteurs, former un vecteur de caractéristiques opérationnelles, et détecter une anomalie si le vecteur est significativement dissemblable de vecteurs précédemment construits.

La demanderesse a également déposé une demande de brevet européenne publiée sous le numéro EP 3 112 960. Cette demande de brevet divulgue une méthode de détection d'anomalie, consistant dans un premier temps à pré-détecter des anomalies, à partir d'une classification de résidus sur des fenêtre temporelles, puis, pour les éléments du réseau pré-classifiés comme anormaux, une classification de ces éléments basée sur une modification itérative de leur caractéristique, jusqu'à ce qu'une simulation du fonctionnement du réseau soit suffisamment proche des mesures observées.

Cette méthode permet une détection fine et efficace d'anomalies sur un réseau de distribution d'eau, car la détection d'anomalies s'effectue en comparant un vecteur avec des vecteurs transformés issus des mêmes capteurs sur un même réseau de distribution d'eau. Ceci permet d'obtenir une détection complètement adaptée aux caractéristiques du réseau de distribution d'eau étudié. Par exemple, un système aquatique constitué de différentes ressources pourra usuellement voir alterner aux points de consommation des eaux ayant une composition différente (conductivité, température, pH ...). Dans un tel système, une anomalie pourra être détectée si une combinaison significativement différente des combinaisons usuelles de ces paramètres apparait, reflétant un nouveau mélange d'eau. De plus, la sensibilité de la méthode peut être définie, pour les différents types d'événements et de mesures.

Cependant, cette méthode peut nécessiter un temps d'entraînement long avant de présenter une caractérisation d'anomalies pleinement opérationnelle. En effet, une détection et une identification d'un type particulier d'anomalie sur la base de cette méthode nécessiterait que plusieurs vecteurs représentatifs de cette anomalie aient été construits et labellisés, et donc que l'anomalie se soit déjà produite, dans ce système aquatique, à plusieurs reprises. Une telle méthode ne serait donc pleinement efficace pour détecter les anomalies dans un système aquatique donné qu'au bout d'une durée suffisamment importante (pouvant aller jusqu'à plusieurs années) pour que toutes les anomalies recherchées se soient déjà produites à plusieurs reprises dans le système aquatique étudié.

Il y a donc besoin d'une détection d'anomalies dans un réseau de distribution d'eau, permettant de détecter et caractériser de manière fine les événements affectant la qualité de l'eau, qui puisse détecter et caractériser rapidement un ensemble d'anomalies visées dans un nouveau système aquatique, disposant de peu de données historiques.

### RESUME DE L'INVENTION

A cet effet, l'invention décrit un dispositif apte à détecter et caractériser des d'anomalies dans un continuum d'eau, comprenant : au moins un lien de communication, respectivement vers au moins un capteur d'au moins une grandeur physique dans le continuum d'eau ; un processeur configuré pour : recevoir des mesures de l'au moins un capteur par l'au moins un lien de communication ; générer une pluralité de fenêtres temporelles des mesures ; pour chaque fenêtre temporelle de ladite pluralité : obtenir un ensemble de valeurs transformées par au moins une transformation des mesures sur la fenêtre temporelle ; appliquer un détecteur d'anomalies à l'ensemble de valeurs transformées afin de détecter un état normal ou anormal de la fenêtre temporelle, ledit détecteur d'anomalies étant paramétré à partir d'ensembles de valeurs transformées issues de l'application de l'au moins une transformation à des fenêtres temporelles de mesures précédentes de l'au moins un capteur ; détecter une anomalie dans le continuum d'eau, en fonction d'un nombre de fenêtres temporelles présentant un état anormal ; exécuter sur les mesures un ensemble de règles prédéfinies de détection d'une variation d'une au moins des grandeurs physiques ; si une anomalie dans le continuum d'eau est détectée, affecter à l'anomalie un type d'anomalies associé à une variation d'un sous-ensemble prédéfini des grandeurs physiques, si la variation de l'une au moins des grandeurs du sous-ensemble est détectée.

Ceci permet une détection efficace des anomalies dans le continuum d'eau. En effet, le détecteur est paramétré à partir de l'apprentissage sur des mesures issues des mêmes capteurs dans le même continuum d'eau. Ainsi, le détecteur d'anomalies est paramétré à partir de mesures prenant en compte les particularités du continuum d'eau, ce qui permet une détection optimisée de l'occurrence de situations anormales.

Une anomalie est typée, une fois détectée, à partir de règles prédéfinies. Ceci permet d'identifier, à partir de règles générales, quel serait le type d'anomalie une fois l'anomalie détectée. Ceci permet donc d'identifier le type de l'anomalie, même si cette anomalie n'a pas encore été détectée dans le continuum d'eau avec les mesures de l'au moins un capteur.

Le dispositif de l'invention permet donc d'obtenir en même temps une détection fine, basée sur un paramétrage spécifique au continuum d'eau, de l'occurrence d'une situation anormale, et un typage général de l'anomalie basé sur une connaissance globale des effets des différentes anomalies, même si l'anomalie n'a pas été détectée dans le continuum d'eau.

Avantageusement, une des transformations des mesures sur une fenêtre temporelle est une transformation des mesures en valeurs représentatives de la variabilité des mesures au sein de la fenêtre temporelle.

Ceci permet de détecter des anomalies se traduisant par une variation rapide d'une grandeur physique dans le continuum d'eau. Par exemple, une variation rapide de la turbidité peut être représentative de mouvements de particules. Une transformation des mesures en valeurs représentatives de la variabilité des mesures de la turbidité au sein de la fenêtre temporelle peut donc permettre d'obtenir des valeurs transformées permettant une détection de ce type d'anomalies.

Avantageusement, la transformation des mesures en valeurs représentatives de la variabilité des mesures au sein de la fenêtre temporelle est réalisée en soustrayant à chaque mesure sur la fenêtre temporelle une médiane desdites mesures sur une fenêtre temporelle glissante comprenant au moins ladite fenêtre temporelle.

Ceci permet de s'affranchir des variations lentes, par exemple à l'échelle d'une journée, des mesures sur une fenêtre temporelle. Cette transformation présente l'avantage, en plus de fournir de manière fiable des valeurs transformées représentatives de la variabilité des mesures au sein de la fenêtre temporelle, d'être simple à mettre en oeuvre et de nécessiter une puissance de calcul réduite pour s'exécuter.

Avantageusement, la transformation ne conserve que des mesures correspondant à un sens de variation prédéfini.

Ceci permet de détecter de manière plus efficace des anomalies se traduisant par une variation rapide des mesures dans un sens donné. Par exemple, des mouvements de particules peuvent se traduire par une augmentation rapide de la turbidité. Ainsi, ne conserver que les mesures correspondant à une augmentation de la turbidité peut permettre de déceler de manière plus sûre cette anomalie.

Avantageusement, une des transformations des mesures sur une fenêtre temporelle consiste en un test de pente sur la fenêtre temporelle.

Ceci permet de détecter des anomalies affectant par des tendances lentes d'une grandeur physique. Par exemple dans un réseau d'eau potable, une augmentation lente du nombre de bactéries couplée ou non à une décroissance lente de la concentration en chlore peut signifier le risque d'apparition d'une anomalie de croissance bactérienne. Il est avantageux d'alerter les opérationnels sur ce type de phénomène en cours pour qu'ils soient suivis et éventuellement corrigés. Dans un système aquatique naturel, une augmentation de la chlorophylle-a sur plusieurs jours entrainant d'abord une augmentation puis une diminution progressive de l'oxygène dissous peut signifier l'apparition de phénomènes de croissances d'algues. Elles risquent de nuire à la vie aquatique et à l'usage du système, et doivent donc être anticipées. Un test de pente sur une fenêtre temporelle permet avantageusement de détecter de telles variations lentes, et donc d'éventuelles anomalies sous-jacentes.

Avantageusement, le test de pente est un test de tendance de Mann-Kendall.

Le test de Mann-Kendall permet une détection efficace de la pente significative.

Avantageusement, au moins deux des capteurs sont des capteurs d'une même grandeur physique en deux points du continuum d'eau, et une des transformations des mesures sur une fenêtre temporelle comprend une différence temporelle entre les mesures des deux capteurs.

Ceci permet de détecter des anomalies se traduisant par des évolutions anormales de grandeurs physiques entre différents points du continuum.

Avantageusement, l'ensemble de valeurs transformées est un vecteur, et le détecteur d'anomalies est une Machine à Vecteurs de Support à une classe.

Ceci permet une détection particulièrement efficace des états anormaux des fenêtres temporelles. En effet, une Machine à Vecteurs de Support à une classe permet de classer de manière particulièrement efficace des vecteurs parmi un ensemble de vecteurs normaux ou anormaux. De plus, une Machine à Vecteur de support à une classe peut être paramétrée par la proportion de vecteurs attendus comme anormaux. Ainsi, le détecteur d'anomalie peut être paramétré facilement, à l'aide de cette proportion, pour détecter plus ou moins de fenêtres temporelles anormales et aider les opérationnels à prioriser les analyses complémentaires et les interventions selon leurs ressources disponibles.

Avantageusement, l'ensemble de valeurs transformées est obtenu par au moins deux transformations des mesures sur la fenêtre temporelle, et le détecteur d'anomalies est configuré pour déterminer un état normal ou anormal de la fenêtre temporelle à partir de l'ensemble des valeurs transformées.

Ceci permet au détecteur d'anomalies de détecter à quel point un ensemble de valeurs transformées est différent des ensembles précédemment recueillis, à partir de l'ensemble des valeurs, et donc de détecter d'éventuels liens entre valeurs transformées issues de grandeurs physiques et/ou de capteurs différents. Cette solution est particulièrement performante dans un réseau de distribution d'eau. En effet, ceci permet de détecter des variations simultanées anormales de plusieurs grandeurs physiques et/ou en plusieurs points d'un réseau de distribution d'eau.

Avantageusement, l'ensemble de valeurs transformées est obtenu par au moins deux transformations des mesures sur la fenêtre temporelle, et le détecteur d'anomalies est configuré pour : détecter des états normaux ou anormaux d'au moins deux sous-ensembles de valeurs transformées respectivement par lesdites au moins deux transformations des mesures sur la fenêtre temporelle ; détecter l'état normal ou anormal de la fenêtre temporelle à partir d'une combinaison desdits états normaux ou anormaux des sous-ensembles.

Ceci permet de détecter dans un premier temps l'état normal ou anormal des valeurs issues de chacune des transformations, par exemple de chaque grandeur physique et/ou capteur. L'état normal ou anormal de l'ensemble de valeurs transformées est défini en fonction des états normaux ou anormaux de chaque sous-ensemble. Ceci permet de prendre en compte de manière plus fine les mesures de chaque grandeur physique et/ou chaque capteur. Cette solution est particulièrement efficace en milieu naturel. En effet, un capteur en milieu naturel est plus susceptible de produire des faux positifs : la variabilité des valeurs des grandeurs physiques y est plus importante, et les capteurs sont susceptibles de subir des dérives lentes dues par exemple à l'encrassement, car les eaux naturelles sont plus chargées en matières en suspension. Les capteurs en milieu naturels sont donc plus susceptibles d'être défaillants et/ou de produire des mesures bruitées. Effectuer une détection d'anomalie sur les mesures issues de chaque transformation (et donc de chaque capteur), puis ne détecter de fenêtre anormales que si au moins deux sous-ensembles présentent des valeurs anormales permet de ne détecter d'anomalies que les mesures issues d'au moins deux capteurs présentent des anomalies.

Avantageusement, le processeur est configuré pour calculer une intensité de variation des grandeurs physiques, et l'ensemble de règles prédéfinies de caractérisation comprend une règle prédéfinie de détection d'une variation de d'une grandeur physique, si l'intensité de variation de ladite grandeur physique sur les mesures est supérieure à un seuil de variation normale.

Ceci permet de détecter efficacement une variation d'une grandeur physique.

Avantageusement, le processeur est configuré pour affecter un indicateur de criticité à l'anomalie, en fonction des intensités des variations des grandeurs physiques du sous-ensemble.

Ceci permet de déterminer automatiquement la gravité de l'anomalie, et donc d'engager les actions correctrices les plus appropriées.

Avantageusement, la détection de la variation de la grandeur physique ne détecte une variation que si la variation de la grandeur physique est conforme à un sens de variation.

Ceci permet, dans les cas où une anomalie est liée à un sens de variation donné (augmentation ou diminution), de ne retenir une variation de la grandeur physique que si la grandeur physique varie dans le sens défini.

Avantageusement, le processeur est configuré, si une anomalie est détectée, pour affecter à l'anomalie l'un au moins des types suivants: un type « croissance bactérienne », en cas de variation de grandeurs physiques d'un sous-ensemble comprenant : une diminution de la concentration en chlore, une augmentation de température, une augmentation de la teneur en carbone organique total, une augmentation de l'absorbance de lumière Ultraviolette de longueur d'onde 254 nm, une augmentation du nombre de bactéries ; un type « mélange d'eau », en cas de variations de grandeurs physiques d'un sous-ensemble comprenant une conductivité, un pH ; une température ; un type « eaux colorées », en cas de variations les grandeurs physiques d'un sous-ensemble comprenant une concentration en chlore, un pH, une augmentation de couleur, une augmentation de turbidité, une augmentation de l'absorbance de lumière Ultraviolette de longueur d'onde 254 nm ; une survitesse, en cas d'augmentation anormale de grandeurs physiques parmi un sous-ensemble comprenant la turbidité et les particules.

Ces exemples permettent de détecter de manière fiable les types d'anomalies susmentionnés.

Avantageusement, le processeur est configuré, si la sortie des de règles prédéfinies de détection d'une variation d'une au moins des grandeurs physiques ne permet pas l'affectation d'un type à une anomalie, pour affecter à cette anomalie un type inconnu.

Ceci permet de générer dans tous les cas une anomalie associée à un type.

Avantageusement, le dispositif comprend une interface (250) pour afficher l'anomalie et son type à un opérateur.

Ceci permet à un opérateur de visualiser les anomalies, et prévoir le cas échéant des opérations correctrices.

Avantageusement, l'interface est configurée pour recevoir de l'opérateur un label concernant l'anomalie ; la fenêtre temporelle de valeurs, et le label concernant l'anomalie sont ajoutés aux données d'entraînement.

Ceci permet de corriger une éventuelle détection incorrecte d'anomalies, et d'améliorer l'entraînement de machines d'apprentissage automatique pour la détection d'anomalies.

L'invention décrit également une méthode de détection et de caractérisation d'anomalies dans un continuum d'eau, comprenant : la réception (810) de mesures d'une pluralité de grandeurs physiques issues d'une pluralité de capteurs de la pluralité de grandeurs physiques dans le continuum d'eau ; la génération (820) d'une pluralité de fenêtres temporelles de mesures ; pour chaque fenêtre de ladite pluralité : l'obtention d'un ensemble de valeurs transformées par au moins une transformation (830) des mesures sur la fenêtre temporelle ; la détection (840) d'un état normal ou anormal de la fenêtre temporelle, ladite détection étant paramétré à partir d'ensembles de valeurs transformées issues de l'application de l'au moins une transformation à des fenêtres temporelles de mesures précédentes de l'au moins un capteur ; la détection (850) d'une anomalie dans le continuum d'eau, en fonction d'un nombre de fenêtres temporelles présentant un état anormal ; l'exécution (860) d'un ensemble de règles prédéfinies de détection d'une variation d'une au moins des grandeurs physiques ; si une anomalie dans le continuum d'eau est détectée, l'affectation (870) à l'anomalie un type d'anomalies associé à une variation d'un sous-ensemble prédéfini des grandeurs physiques, si la variation de l'une au moins des grandeurs du sous-ensemble est détectée.

L'invention décrit également un produit programme d'ordinateur comprenant des instructions de code de programme enregistrés sur un support lisible par un ordinateur comprenant un processeur pour la détection d'anomalies dans un continuum d'eau, ledit programme d'ordinateur comprenant des moyens de programmation lisibles par ordinateur pour: recevoir des mesures d'une pluralité de grandeurs physiques issues d'une pluralité de capteurs de la pluralité de grandeurs physiques dans le continuum d'eau ; générer une pluralité de fenêtres temporelles de mesures ; pour chaque fenêtre de ladite pluralité : obtenir un ensemble de valeurs transformées par au moins une transformation des mesures sur la fenêtre temporelle ; appliquer un détecteur d'anomalies à l'ensemble de valeurs transformées afin de détecter un état normal ou anormal de la fenêtre temporelle, ledit détecteur d'anomalies étant paramétré à partir d'ensembles de valeurs transformées issues de l'application de l'au moins une transformation à des fenêtres temporelles de mesures précédentes de l'au moins un capteur ; détecter une anomalie dans le continuum d'eau, en fonction d'un nombre de fenêtres temporelles présentant un état anormal ; exécuter un ensemble de règles prédéfinies de détection d'une variation d'une au moins des grandeurs physiques ; si une anomalie dans le continuum d'eau est détectée, affecter à l'anomalie un type d'anomalies associé à une variation d'un sous-ensemble prédéfini des grandeurs physiques, si la variation de l'une au moins des grandeurs du sous-ensemble est détectée.

### LISTE DES FIGURES

D'autres caractéristiques apparaîtront à la lecture de la description détaillée donnée à titre d'exemple et non limitative qui suit faite au regard de dessins annexés dans lesquels :
- la figure 1 représente un exemple de sonde destinée à détecter des anomalies dans réseau de distribution d'eau, selon un ensemble de modes de mise en oeuvre de l'invention ;
- la figure 2 représente un exemple de dispositif pour détecter des anomalies dans un continuum d'eau selon un ensemble de modes de mise en oeuvre de l'invention ;
- la figure 3 représente un exemple de transformation de mesures d'une grandeur physique dans un mode de mise en oeuvre de l'invention ;
- la figure 4 représente un exemple de détection d'une fenêtre temporelle anormale, dans un réseau de distribution d'eau potable ;
- la figure 5 représente un exemple de détection d'une fenêtre temporelle anormale, dans de l'eau en milieu naturel ;
- les figures 6a et 6b représentent deux exemples de combinaisons d'événements sur un réseau de distribution d'eau, dans un ensemble de modes de mise en oeuvre de l'invention ;
- la figure 7 représente une interface de présentation d'événements survenant dans un réseau de distribution d'eau à un opérateur dans un ensemble de modes de mise en oeuvre de l'invention ;
- la figure 8 représente une méthode de détection et de caractérisation d'anomalies dans un continuum d'eau selon l'invention.

### DESCRIPTION DETAILLEE

Dans la suite de la description le procédé selon l'invention est principalement illustré par des exemples relatifs à la détection d'anomalies dans un réseau de distribution d'eau potable. Cependant, l'invention n'est pas restreinte à ces exemples, et peut être appliquée à toute détection d'événement lié à la qualité de l'eau, à partir de mesures d'au moins deux capteurs.

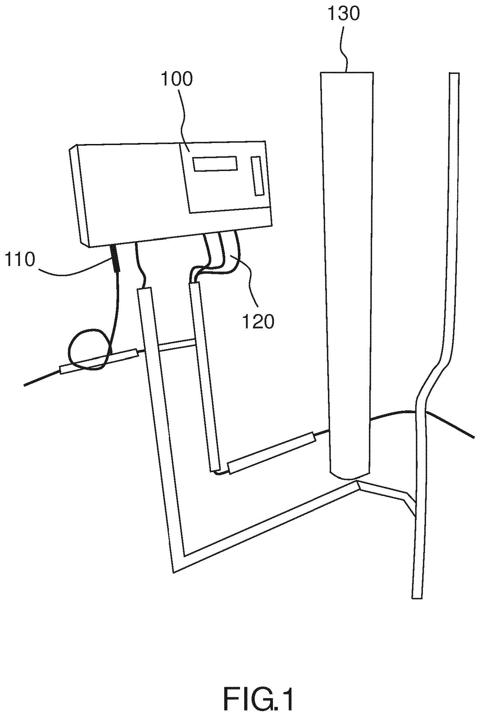

La figure 1 représente un exemple de sonde destinée à détecter des anomalies dans réseau de distribution d'eau, selon un ensemble de modes de réalisation de l'invention.

La sonde 100 destinée à détecter des anomalies dans un réseau de distribution d'eau. La sonde peut être placée à n'importe quel emplacement d'un réseau de distribution d'eau, par exemple en sortie d'une usine de production d'eau potable, en sortie de réservoir, à un point de consommation, où en tout autre point.

La sonde 100 est connectée à des canalisations 130 du réseau de distribution d'eau, par exemple par un ensemble de câbles de capteurs 120 ou directement connectée sur le réseau (insertion), et est alimentée en électricité 110 ou sur batterie.

La sonde 100 peut comprendre un ou plusieurs capteurs de grandeurs physiques du réseau de distribution d'eau. Par exemple, la sonde 100 peut comprendre un ou plusieurs capteurs choisis parmi des capteurs de concentration en chlore, température, TOC (de l'anglais Total Organic Carbon, signifiant Carbone Organique Total), UV 254 (absorbance de l'eau pour une lumière ultraviolette de longueur d'onde 254 nm), conductivité, pH, couleur, turbidité, nombre de particules, nombre de bactéries, oxygène dissous, chlorophylle a ou tout capteur d'une grandeur physique pouvant caractériser l'eau.

La sonde 100 permet ainsi, dans un ensemble de modes de réalisation de l'invention, d'effectuer des mesures d'un ensemble de paramètres représentatifs de la qualité de l'eau en un point du réseau de distribution d'eau.

Dans un ensemble de modes de réalisation de l'invention, la sonde 100 comprend des moyens des communications afin de transmettre les mesures des capteurs embarqués. Par exemple, la sonde peut comprendre une connexion filaire ou radio vers un serveur afin d'envoyer les mesures à un serveur configuré pour détecter des anomalies dans le réseau de distribution d'eau. La sonde 100 peut ainsi être couplée à un capteur intelligent de consommation d'eau envoyant des données de consommation en télé-relève, en envoyant, de manière combinée, des données de consommation d'eau et mesures des capteurs.

Dans un ensemble de modes de réalisation de l'invention, la sonde comprend un processeur configuré pour détecter et caractériser, à partir des mesures des capteurs, des anomalies dans le réseau de distribution d'eau.

Des exemples de détection et de caractérisation d'anomalies par un processeur seront données ci-dessous, les techniques de détection et de caractérisation d'anomalies décrites en références aux figures suivantes étant applicables à un processeur embarqué dans la sonde 100.

Bien que la sonde 100 représente un exemple de sonde dans un réseau de distribution d'eau, de telles sondes peuvent également être déployées dans de l'eau en milieu naturel, par exemple dans un lac, étang, rivière ou dans tout autre système aquatique, et ceci éventuellement à différentes profondeurs.

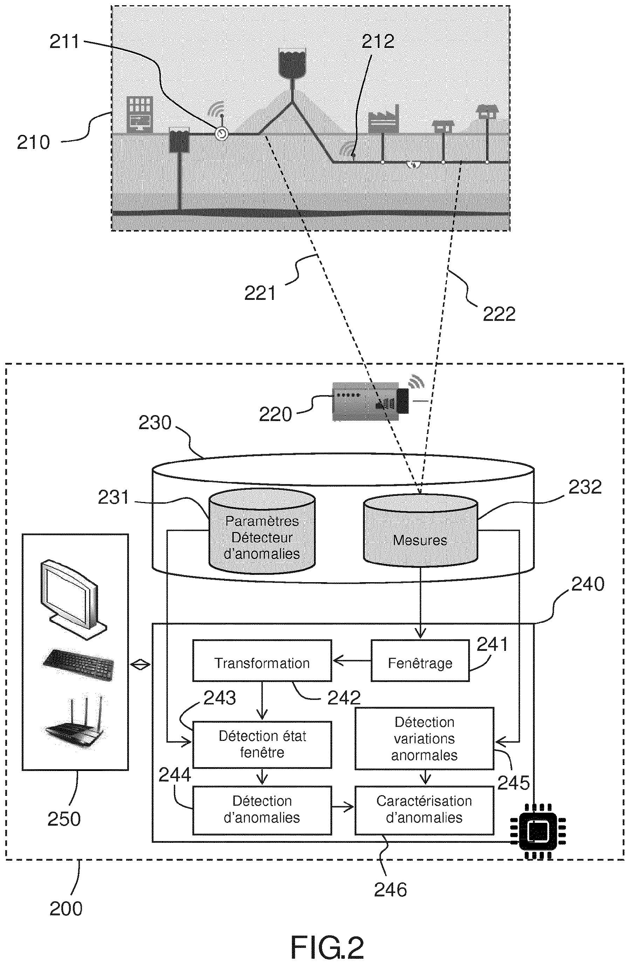

**La** figure 2 représente un exemple de dispositif pour détecter des anomalies dans un continuum d'eau.

Le réseau 210 est un réseau de distribution d'eau équipé d'au moins un capteur 211, 212 d'au moins une grandeur physique. Par exemple, les capteurs 211, 212 peuvent comprendre un ou plusieurs capteurs choisis parmi des capteurs de concentration en chlore, température, TOC, UV 254, conductivité, pH, couleur, turbidité, nombre de particules, nombre de bactéries, oxygène dissous, chlorophylle a ou tout capteur d'une grandeur physique pouvant caractériser l'eau. Les capteurs 211, 212 peuvent être aussi bien des capteurs isolés, que des capteurs localisés au sein de sondes multi capteurs, comme par exemple la sonde 100 représentée en figure 1.

Bien que la figure 2 représente des capteurs dans un réseau de distribution d'eau, l'invention est également applicable à des capteurs d'eau en milieu naturel, par exemple dans un lac, un étang, une rivière, ou de manière plus générale dans un système aquatique. Les capteurs peuvent être situés au même endroit, ou répartis dans plusieurs endroits du réseau de distribution d'eau ou du milieu naturel. Cependant, les capteurs doivent être situés dans le même continuum d'eau, c'est-à-dire que, si les capteurs ne sont pas situés au même endroit, ils doivent être situés dans un même système aquatique ou des systèmes aquatiques communicants entre eux, de manière à ce que, lorsque plusieurs capteurs sont présents, leurs mesures puissent être recoupées pour détecter et caractériser les anomalies dans un même continuum d'eau. Cette condition est remplie si les capteurs sont situés dans un même réseau de distribution d'eau, en deux points d'un lac ou d'une rivière, dans un lac et une rivière affluant ou effluant de ce lac, ou plus généralement en deux points d'un même continuum d'eau. Le terme « continuum d'eau » peut donc aussi bien désigner un réseau de distribution d'eau qu'un système aquatique en milieu naturel.

Le dispositif 200 permet de détecter et caractériser des d'anomalies dans un continuum d'eau, par exemple le réseau de distribution d'eau 210. Il comprend à cet effet un processeur 240 et au moins un lien 221 et 222 avec les capteurs 211, 212. Dans un ensemble de modes de réalisation de l'invention, la communication avec les capteurs 211, 212 peut s'effectuer par un système d'acquisition de mesures, par exemple une plateforme SCADA (Supervisory Control And Data Acquisition). Dans un ensemble de modes de réalisation de l'invention, les liens de communication 221, 222 sont une liaison radio avec un récepteur 220. De nombreuses autres implémentations des liens de communication sont possibles. Par exemple, les capteurs 220, 221 peuvent envoyer des valeurs à un concentrateur, qui est connecté par une liaison radio ou une liaison téléphonique cellulaire à un modem dans le dispositif 200. Certaines parties du lien de communication peuvent être constituées par des liaisons filaires. L'homme du métier peut définir, sans effort, les liens de communication pertinents pour un cas d'utilisation donné, par exemple en sélectionnant l'une des liaisons de communication bien connues dans le domaine de la gestion de réseaux de distribution d'eau. Selon différents modes de réalisation de l'invention, le dispositif 200 peut appartenir à différents types de dispositifs informatiques. Par exemple, le dispositif 200 peut être un ordinateur personnel, une station de travail, un serveur, une tablette numérique, ou tout autre dispositif adapté.

Le système 200 comprend en outre un support de stockage 230. Dans l'exemple représenté sur la figure 2, le support de stockage est situé dans le dispositif 200. Dans d'autres modes de réalisation de l'invention, les supports de stockage peuvent être situés à l'extérieur du dispositif informatique. Par exemple, il peut s'agir d'un disque dur partagé, ou d'une base de données distante, accessible à travers des requêtes par le dispositif informatique.

Dans l'exemple représenté sur la figure 2, le support de stockage comprend des paramètres 231 d'un détecteur d'anomalies dans le continuum d'eau, une base de données de mesures 232. Les paramètres 231 permettent de paramétrer un détecteur d'anomalies dans le continuum d'eau. Comme il sera décrit plus en détail ci-après, les paramètres sont obtenus à partir de transformations de mesures précédentes. Par exemple, les paramètres 231 peuvent être des vecteurs de mesures précédentes transformées, ou une configuration d'un moteur d'apprentissage automatique obtenue à l'aide de vecteurs de mesures précédentes transformées. La base de données de mesures 232 est utilisée pour stocker des mesures provenant des capteurs 211, 212. Les mesures comprennent notamment des valeurs de grandeurs physiques mesurées par le ou les capteurs 211, 212, et un horodatage avec la date / heure des mesures. Le support de stockage 230 peut par exemple être un disque dur, un lecteur à état solide, une mémoire flash ou tout autre type de stockage connu. Dans d'autres modes de réalisation, les paramètres 231 et/ou la base de données de mesures 232 peuvent être stockés sur différents supports de stockage, à l'intérieur ou à l'extérieur du dispositif informatique 200.

Le dispositif 200 peut également comprendre un ensemble d'entrées/sorties 250 telles qu'un écran, un clavier ou un routeur.

Le dispositif 200 comprend un processeur 240. Différents types de processeur sont utilisables dans le cadre de l'invention : le processeur peut être par exemple un microprocesseur, un microcontrôleur ou un processeur de signal numérique (également appelé en anglais Digital Signal Processor ou DSP). Le processeur n'est limité à aucun type ou architecture de processeur, et peut être configuré pour exécuter des opérations en chargeant des éléments de code exécutables. Le processeur peut être aussi bien situé dans une sonde comprenant l'au moins un capteur que dans un ordinateur personnel ou serveur distant.

Le processeur est configuré pour recevoir des mesures de l'au moins un capteur 211, 212. Dans l'exemple de la figure 2, le processeur est configuré pour lire les mesures dans la base de données de mesures 232. Cependant, le processeur 240 peut également recevoir les mesures d'une autre manière par exemple en recevant directement les mesures des capteurs et en les stockant dans une mémoire de travail locale.

Le processeur 240 est de plus configuré pour générer une pluralité de fenêtres temporelles de mesures. Selon différents modes de réalisation de l'invention, les fenêtres temporelles peuvent être aussi bien des fenêtres temporelles successives, que des fenêtres successives se recoupant.

De nombreuses durées de fenêtres sont possibles. Par exemple, les fenêtres temporelles peuvent avoir des durées de 5min, 15min, 1h, 6h, 12h ou 24h.

Les mesures peuvent être échantillonnées à intervalle réguliers au sein d'une fenêtre. Par exemple, l'ensemble des capteurs peut être échantillonnés à la même fréquence, et donc produire des mesures de manière simultanée. Dans d'autres cas, les fréquences d'échantillonnages peuvent être variables. Par exemple, certains capteurs peuvent être échantillonnés toutes les 5 minutes quand d'autres le sont toutes les 15 minutes. Il est alors possible d'uniformiser les pas de temps à l'aide d'interpolation des données. Les mesures sont ainsi synchronisées en prenant en compte des pas de temps réguliers à la plus petite durée d'acquisition disponible. Ceci permet d'avoir les mêmes pas de temps pour tous les capteurs, échantillonnés à la fréquence d'acquisition la plus élevée.

Le processeur est configuré, pour chaque fenêtre temporelle, pour obtenir un ensemble de valeurs transformées par au moins une transformation 242 des mesures sur la fenêtre temporelle.

L'au moins une transformation 242 permet d'obtenir des valeurs transformées, dont certaines propriétés peuvent être caractéristiques d'anomalies.

Par exemple, une transformation 242 peut consister en une suppression des tendances longues (typiquement journalières ou hebdomadaires) pour ne conserver que les variations rapides des mesures d'une grandeur physique, c'est-à-dire que la transformation 242 transforme les mesures en valeurs représentatives de la variabilité des mesures au sein de la fenêtre temporelle : plus les valeurs transformées sont élevées, plus la fenêtre temporelle est affectée de variations rapides, et plus les mesures sont dispersées. Par exemple, une des transformations des mesures sur une fenêtre peut consister en un filtrage des variations rapides, par exemple de l'ordre de quelques heures, par rapport à la tendance journalière, équivalent à un filtre passe-haut, des mesures d'une grandeur physique. Une telle transformation permet de ne conserver que les variations rapides des mesures d'une grandeur physique, et donc de détecter des anomalies liées à une variation anormalement rapide de cette grandeur. Par exemple des mouvements de sédiments peuvent être détectés grâce à des variation anormalement rapides de la turbidité.

La transformation des les mesures en valeurs représentatives de la variabilité des mesures au sein de la fenêtre temporelle peut être réalisée en soustrayant à chaque mesure sur la fenêtre temporelle une médiane desdites mesures sur la fenêtre temporelle ou sur une fenêtre temporelle plus longue. Ceci permet un filtrage passe-haut efficace, tout en conservant l'information donnée par chaque mesure. A titre d'alternative, le filtrage passe-haut peut également être réalisé par d'autres moyens. Par exemple, il peut être réalisé en soustrayant à chaque mesure sur la fenêtre temporelle une moyenne desdites mesures sur la fenêtre temporelle, ou en effectuant une transformée fréquentielle.

La transformation peut en outre ne conserver que des mesures correspondant à un sens de variation prédéfini. Par exemple, dans le cas de mesures de turbidité, on pourra ne conserver que des augmentations brusques, qui peuvent être causées par des mouvements de sédiments. Dans le cas de mesures de concentration en chlore, on pourra ne conserver que les mesures représentatives d'une diminution de la concentration en chlore, pouvant caractériser un accroissement du nombre de bactéries ou du taux de matière organique. Ainsi, ne conserver que les mesures correspondant à une augmentation de la turbidité peut permettre de déceler de manière plus sûre cette anomalie. Ceci peut s'effectuer par exemple, dans le cas évoqué précédemment où le filtrage des variations rapides est réalisé en soustrayant à chaque mesure sur la fenêtre temporelle une médiane desdites mesures sur la fenêtre temporelle, en ne conservant après soustraction :
- que les valeurs positives ou nulles, si le sens de variation recherché est une augmentation ;
- que les valeurs négatives ou nulles, si le sens de variation recherché est une diminution.

Une transformation peut également consister en l'application d'un test de pente sur une fenêtre temporelle. Ceci permet de détecter les variations lentes sur une fenêtre temporelle. Certaines anomalies peuvent générer des variations lentes de certaines grandeurs physiques. Par exemple dans un réseau d'eau potable, une augmentation lente du nombre de bactéries couplée ou non à une décroissance lente de la concentration en chlore peut signifier le risque d'apparition d'une anomalie de croissance bactérienne. Il est avantageux d'alerter les opérationnels sur ce type de phénomène en cours pour qu'ils soient suivis et éventuellement corrigés. Dans un système aquatique naturel, une augmentation de la chlorophylle-a sur plusieurs jours entrainant d'abord une augmentation puis une diminution progressive de l'oxygène dissous peut signifier l'apparition de phénomènes de croissances d'algues. Elles risquent de nuire à la vie aquatique et à l'usage du système, et doivent donc être anticipées.

La taille de la fenêtre temporelle peut être adaptée à la durée estimée des phénomènes en jeu. Par exemple, un test de pente peut s'effectuer en comparant les médianes des mesures par jour, sur une fenêtre temporelle de 5 jours successifs. Le test de pente peut être par exemple un test de tendance de Mann-Kendall, particulièrement fiable. Un coefficient de pente peut être estimé par exemple grâce à un estimateur de Theil-Sen.

Les transformations évoquées ci-dessus sont appliquées à des fenêtres temporelles de mesures d'une grandeur physique issues d'un seul capteur. Cependant, l'invention n'est pas restreinte à ces exemples. Par exemple, l'homme du métier pourrait définir des transformations permettant d'établir un lien entre des mesures d'une même grandeur physique issues de deux capteurs différents, afin d'obtenir des valeurs transformées représentatives de l'évolution d'une grandeur physique dans le continuum d'eau. Par exemple, deux capteurs d'oxygène dissous peuvent être situés en amont et en aval d'une rivière, et une transformation conjointe des mesures issues des deux capteurs permet d'obtenir des valeurs transformées caractérisant l'évolution de l'oxygène dissous dans la rivière.

Par exemple, au moins deux des capteurs peuvent être des capteurs d'une même grandeur physique en deux points distincts du continuum d'eau, et une des transformations des mesures sur une fenêtre temporelle peut comprendre une différence temporelle entre les mesures des deux capteurs.

Dans de nombreux cas, l'écoulement de l'eau dans le continuum définit des corrélations temporelles entre les grandeurs physiques en différents points du continuum. Par exemple, si le continuum d'eau est une rivière, et que deux capteurs de conductivité sont situés, l'un en aval de l'autre, les mesures issues du capteur en aval seront corrélées avec les mesures du capteur en amont, moyennant une différence temporelle correspondant au temps moyen mis par l'eau pour parcourir la distance entre le capteur en amont et le capteur en aval.

Un décalage temporel optimal entre les mesures de deux capteurs d'une même grandeur physique en deux points du continuum peut être estimé à partir des données historiques, en effectuant sur les mesures précédentes des corrélations croisées entre les mesures des deux capteurs, en effectuant un test de significativité de la corrélation, et en sélectionnant le pic le plus important.

Lorsqu'une transformation des mesures consiste à effectuer une différence temporelle selon le décalage temporel optimal entre les mesures de deux capteurs d'une même grandeur physique en deux points distincts du continuum, les valeurs transformées seront faibles si l'écoulement est normal, et si aucun événement n'impactant la grandeur physique testée ne s'est produit entre les deux capteurs.

Au contraire, des valeurs élevées pourraient être représentatives :
- soit d'une modification de l'écoulement de l'eau dans le continuum, impliquant que le temps mis par l'eau pour parcourir l'espace entre les deux capteurs devient différent du décalage temporel optimal préalablement calculé, les mesures de la grandeur physique n'étant alors plus corrélées selon ce décalage ;
- soit d'un événement affectant la grandeur physique, survenant entre les deux capteurs.

Une telle transformation permet donc non seulement de détecter une anomalie à partir de mesures distribuées dans le réseau, mais également de fournir des informations de localisation d'une éventuelle anomalie.

La ou les transformations ainsi définies permet d'obtenir des valeurs transformées caractéristiques, également appelées « features », permettant de détecter des anomalies. Selon différents modes de réalisation de l'invention, différentes transformations peuvent être appliquées sur une même fenêtre temporelle à des mesures de différentes grandeurs physiques. Il est également possible d'appliquer plusieurs transformations à une même grandeur physique. L'homme de l'art peut ainsi, connaissant les caractéristiques associées à de potentielles anomalies dans le continuum d'eau, définir les transformations les plus adaptées aux types de mesures disponibles, et aux anomalies recherchées.

L'ensemble de valeurs transformées peut prendre de nombreuses formes, à partir du moment où la structure des valeurs transformées sur les différentes fenêtres temporelles reste identiques, afin de permettre une identification des ensembles anormaux. Il est nécessaire que les transformations soient appliquées de manière similaire aux données du vecteur courant et aux vecteurs passés, servant de données d'entraînement pour la détection d'anomalies.

Par exemple, l'ensemble de valeurs transformées peut être obtenu sous la forme d'un vecteur. Si une seule transformation est appliquée, le vecteur peut simplement comprendre les valeurs transformées sur les différents paramètres sur la fenêtre temporelle. Il peut également contenir les mesures successives transformées sur la fenêtre temporelle. Si plusieurs transformations sont appliquées, les valeurs transformées à l'issue de chaque transformation peuvent être concaténées afin de former un vecteur unique.

Dans un ensemble de modes de réalisation de l'invention, le processeur est configuré pour normaliser l'ensemble de valeurs transformées, afin de détecter des anomalies sur des valeurs homogènes. Par exemple, le processeur peut être configuré pour calculer l'écart-type des valeurs transformées sur une fenêtre temporelle, et diviser toutes les valeurs de la fenêtre par cet écart-type. Le processeur peut également être configuré pour supprimer les valeurs extrêmes singulières, dans l'optique de calculer les features ainsi que les indicateurs statistiques (écart-type, moyenne, médiane) utilisé pour la normalisation. Plusieurs modes de réalisations sont possibles à cet effet. Par exemple, le processeur peut être configuré pour calculer la moyenne et l'écart-type des valeurs sur la fenêtre, et supprimer les valeurs supérieures d'un nombre prédéfini de fois l'écart-type à la moyenne, et/ou les valeurs inférieurs de ce même nombre prédéfini de fois à la moyenne, par exemple les valeurs supérieures à la moyenne plus trois fois l'écart-type, ou inférieures à la moyenne moins trois fois l'écart-type. Une autre option consiste à supprimer un pourcentage donné (par exemple 5%) des valeurs transformées les plus élevées et/ou les plus basses sur la fenêtre temporelle.

Lorsque l'ensemble de valeurs transformées est obtenu en concaténant des valeurs transformées issues de plusieurs transformations, la normalisation des valeurs peut être effectuée séparément sur les valeurs issues de chaque transformation. Ceci permet d'obtenir un ensemble de valeurs transformées homogènes, même si les transformations appliquées, et les amplitudes des mesures initiales sont très différentes. Il n'existe alors pas de paramètre dominant les autres de manière intrinsèque dans l'espace d'apprentissage.

Dans un ensemble de modes de réalisations de l'invention, l'ensemble de valeurs transformées peut également comprendre des données conjoncturelles pouvant avoir un impact sur les grandeurs physiques mesurées. Par exemple, ces données peuvent comprendre des données météorologiques telle que des mesures d'hygrométrie, de température de l'air, d'ensoleillement, de cumul de pluie ou un nombre de jours successifs sans pluie au moment où les mesures sont effectuées. Ces paramètres affectent en particulier la qualité des eaux de systèmes aquatiques naturels. Les données de cumul de pluie sont par exemple utilisées, via un seuil, pour retirer les pas de temps correspondant à un temps de pluie de la détection d'anomalies.

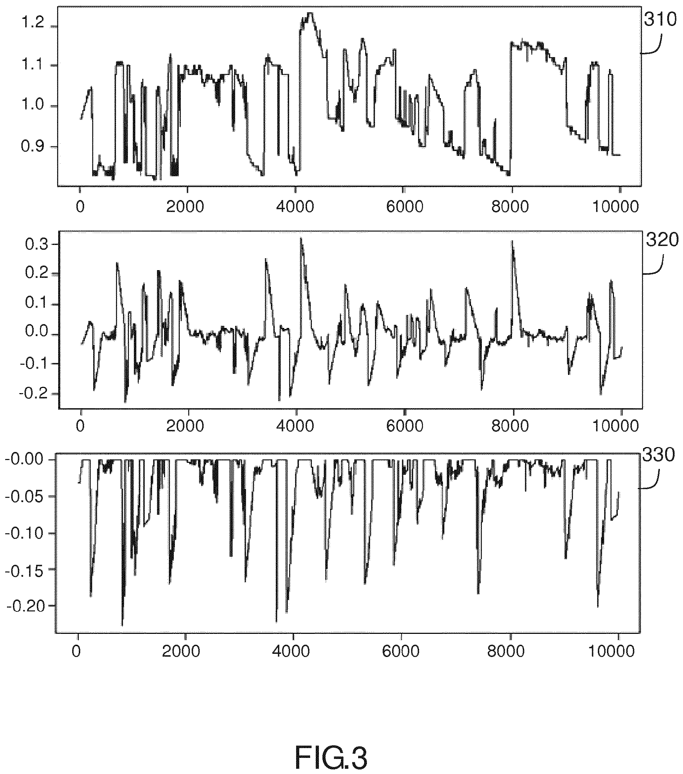

La figure 3 représente un exemple de transformation de mesures d'une grandeur physique dans un mode de réalisation de l'invention.

La courbe 310 représente une fenêtre temporelle de mesures de concentration en chlore. L'axe horizontal représente le temps écoulé, en nombre de pas de temps de 5 minutes. L'axe vertical représente les intensités de mesures, en ppm (parties par million).

Une première étape de transformation consiste à calculer une médiane des mesures sur la fenêtre temporelle, et à soustraire cette médiane aux mesures, afin d'obtenir un premier niveau de transformations 320. Les valeurs transformées sont alors représentatives des variations rapides des mesures.

Une deuxième étape de transformation consiste à ne conserver que les valeurs négatives (donc représentative d'une diminution de concentration en chlore). Les valeurs transformées 330 permettent donc d'évaluer un niveau de diminution rapide de concentration en chlore. Une diminution excessive de concentration en chlore pouvant être causée par un phénomène de croissance bactérienne, ces valeurs transformées peuvent être utilisées pour détecter cette anomalie.

Cette transformation est donnée à titre d'exemple uniquement d'une transformation selon l'invention. L'homme de l'art pourra aisément définir les transformations les plus adaptées en fonction des types d'anomalies recherchées, par exemple en ne conservant, sur une fenêtre temporelle que les variations rapides ou lentes, et que les augmentations ou diminutions d'une grandeur physique donnée.

Revenant à la figure 2, Le processeur est de plus configuré pour appliquer un détecteur d'anomalies 243 à l'ensemble de valeurs transformées afin de détecter un état normal ou anormal de la fenêtre temporelle, ledit détecteur d'anomalies étant paramétré à partir d'ensembles de valeurs transformées issues de l'application de l'au moins une transformation à des fenêtres temporelles de mesures précédentes de l'au moins un capteur.

Le détecteur d'anomalies peut prendre différentes formes. Par exemple, le détecteur d'anomalies peut être un ensemble d'instructions de processeur, un module logiciel, ou un module d'apprentissage automatique configuré à l'aide de détections d'anomalies précédentes. Le détecteur d'anomalies est configuré pour détecter, pour une fenêtre temporelle, si l'ensemble de valeurs est représentatif d'un état anormal de la fenêtre temporelle. A cet effet, le détecteur d'anomalies est paramétré avec des ensembles de valeurs transformées précédentes, issues de l'au moins une transformation 242 à des fenêtres temporelles de mesures précédentes de l'au moins un capteur 211, 212. Ainsi, le détecteur d'anomalies est entraîné avec des valeurs transformées représentatives du continuum d'eau, ce qui permet une détection d'anomalies bien adaptée au continuum d'eau étudié. Bien que le détecteur d'anomalies s'améliore au fur et à mesure que la quantité de données d'apprentissage augmente, un détecteur n'indiquant qu'un état normal ou anormal, sur des données issues des mêmes capteurs, peut être mis en place rapidement. En particulier, un tel détecteur peut détecter des anomalies dès que des valeurs transformées sont significativement différentes des valeurs transformées de la base d'apprentissage. En revanche, il ne nécessite ni d'avoir rencontré toutes les anomalies précédemment, ni que des anomalies aient été labellisées. Ainsi, le détecteur d'anomalies peut être fonctionnel très rapidement, dès que quelques mesures transformées sont disponibles.

La détection d'un état anormal de la fenêtre peut par exemple s'effectuer par le détecteur d'anomalie en affectant un score, par exemple entre 0 et 1, à l'ensemble de valeurs transformées d'une fenêtre indiquant à quel point cet ensemble est différent des ensembles de valeurs transformées précédents, et en déterminant l'état de la fenêtre comme anormal si le score est supérieur à un seuil prédéfini. Il est également possible de détecter un état anormal d'une fenêtre temporelle, si les valeurs transformées font partie d'un pourcentage donné des valeurs transformées les plus différentes des valeurs précédemment recueillies.

De nombreux modes de réalisation du détecteur d'anomalies sont possibles. En particulier, le détecteur d'anomalies peut être un algorithme d'apprentissage machine, apprenant de manière automatique, à partir des valeurs transformées précédemment obtenues, les valeurs transformées représentatives d'un état normal ou anormal d'une fenêtre temporelle.

Dans un ensemble de modes de réalisation de l'invention, l'ensemble de valeurs transformées est un vecteur, et le détecteur d'anomalies est une Machine à Vecteurs de Support à une classe (ou one-class SVM, de l'anglais one-class Support Vector Machine, littéralement Machine à Vecteurs de Support à une classe). Ce type de détecteur d'anomalies permet une détection particulièrement efficace des états anormaux des fenêtres temporelles. En effet, une Machine à Vecteurs de Support à une classe permet de représenter de manière particulièrement efficace la frontière entre vecteurs normaux et anormaux (trop éloignés des normaux), afin de déterminer un niveau de différences entre un vecteur et les vecteurs préalablement étudiés. De plus, une Machine à Machine à Vecteurs de Support à une classe est paramétrée au moyen de la proportion de vecteurs attendus comme anormaux. Un détecteur d'anomalies de type Machine à Vecteurs de Support à une classe présente l'avantage de fournir une classification efficace des données en états normaux ou anormaux. De plus, il peut être mis en place très rapidement, avec une quantité de données d'entraînement limitée.

La Machine à Vecteurs de Support à une classe peut ainsi être paramétrée avec un ratio de vecteur anormaux variable, par exemple compris entre 1/100 et 1/10000 (c'est-à-dire avec un taux d'ensembles de valeurs transformées anormaux compris entre 1/100 et 1/10000, la Machine à Vecteurs de Support à une classe adaptant alors automatiquement sa définition de l'ensemble anormal afin d'atteindre ce ratio de valeurs anormales). Le ratio peut être ajusté à tout moment, pour détecter un plus grand ou plus petit nombre d'ensembles de données comme anormaux.

Dans un ensemble de modes de réalisation de l'invention, l'ensemble de valeurs transformées est obtenu par au moins deux transformations des mesures sur la fenêtre temporelle. C'est par exemple le cas lorsque l'ensemble de valeurs transformées est obtenu en transformant des mesures de plusieurs grandeurs physiques et/ou des mesures issues de plusieurs capteurs. Par exemple, une telle configuration peut être obtenue si l'ensemble de valeurs transformées comprend des valeurs issues d'une transformation de mesures de conductivité, une transformation de mesures de pH et une transformation de mesures de température.

Dans ces cas, peut se poser la question de la manière le plus adéquate de détecter des anomalies sur cet ensemble de valeurs transformées.

Dans un ensemble de modes de réalisation de l'invention, le détecteur d'anomalies est configuré pour déterminer un état normal ou anormal de la fenêtre temporelle à partir de l'ensemble des valeurs transformées.

Ceci permet au détecteur d'anomalies de détecter, à partir de l'ensemble des valeurs transformées issues de grandeurs physiques et/ou de capteurs différents, à quel point cet ensemble est différent des ensembles précédemment recueillis, et donc de détecteur d'éventuels liens entre valeurs transformées issues de grandeurs physiques et/ou de capteurs différents. Cette solution est particulièrement performante dans un réseau de distribution d'eau. Cette solution permet en effet de détecter à la fois des variations importantes sur un seul paramètre ou des variations moins importantes mais s'appliquant à plusieurs paramètres simultanément. La détection peut ainsi mettre en évidence de manière particulièrement fine des phénomènes affectant plusieurs paramètres. Dans le cas des réseaux d'eau potable, la variabilité du signal est en général mieux maîtrisée car due à des opérations métiers régulières. De plus les capteurs sont dans un environnement, l'eau potable, plus propre. Ils subissent généralement moins de dérive.

Dans un ensemble de modes de réalisation de l'invention, l'ensemble de valeurs transformées est obtenu par au moins deux transformations des mesures sur la fenêtre temporelle, et le détecteur d'anomalies est configuré pour :
- détecter des états normaux ou anormaux d'au moins deux sous-ensembles de valeurs transformées respectivement par lesdites au moins deux transformations des mesures sur la fenêtre temporelle ;
- détecter l'état normal ou anormal de la fenêtre temporelle à partir d'une combinaison desdits états normaux ou anormaux des sous-ensembles.

Les différents sous-ensembles correspondent typiquement à des sous-ensembles de mesures issues de chaque capteur et/ou de chaque grandeur physique sur la fenêtre temporelle. Par exemple, si une fenêtre temporelle comprend des mesures de conductivité issues d'un capteur unique, et de turbidité issues de deux capteurs différents, l'ensemble des mesures peut être séparé en trois sous-ensembles : un sous-ensemble de mesures de conductivité, et deux sous-ensembles de mesures de turbidité, correspondant aux mesures issues de chacun des deux capteurs.

Il est également possible de définir des sous-ensembles selon des règles différentes Par exemple, un sous-ensemble pourrait correspondre à des mesures d'une même sonde multi-capteur, ou des mesures d'une même grandeur physique issues de plusieurs capteurs en plusieurs points différents.

Ceci permet de détecter dans un premier temps l'état normal ou anormal des valeurs issues de chacune des transformations, par exemple de chaque grandeur physique et/ou capteur. L'état normal ou anormal de l'ensemble de valeurs transformées est défini en fonction des états normaux ou anormaux de chaque sous-ensemble. Ceci permet de prendre en compte de manière différenciée les mesures de chaque grandeur physique et/ou chaque capteur. Cette solution est particulièrement efficace en milieu naturel. En effet les mesures en milieu naturel sont sujettes à une variabilité plus importante de chaque paramètre, ainsi qu'à des défaillances ou des dérives des capteurs plus fréquentes du fait de l'hostilité du milieu. Il est donc pertinent de chercher à s'assurer que les paramètres présentent des valeurs anormales de manière simultanée ou légèrement décalée dans le temps, pour diminuer les fausses alertes.

Dans des modes de réalisation de l'invention dans lesquels la détection d'anomalies s'effectue à l'aide d'algorithmes d'apprentissage machine, par exemple une Machine à Vecteurs de Support à une classe, un apprentissage séparé peut être réalisé pour chacun des sous-ensembles : les différentes transformations sont appliquées à chacune des fenêtres temporelles de mesures précédentes utilisées pour l'apprentissage, afin d'obtenir, pour chacune de ces fenêtres, les sous-ensembles de valeurs transformées correspondantes. L'apprentissage s'effectue alors séparément, pour chaque sous-ensemble, sur les données d'entraînement ainsi obtenues. Ceci permet une détection plus précise des situations anormales sur un capteur et/ou une grandeur physique, en éliminant une partie des fausses alertes liées aux défauts capteurs ou à la variabilité naturelle plus élevée des paramètres mesurés.

La combinaison des états normaux ou anormaux des sous-ensembles peut se faire de différentes manières. Par exemple, un état anormal de la fenêtre temporelle peut être détecté, si au moins un des sous-ensembles a un état anormal. Ceci permet de détecter une anomalie, si les mesures transformées d'au moins un capteur ou une grandeur physiques sont anormales. Il est également possible de détecter un état anormal de la fenêtre temporelle, seulement si tous les sous-ensembles présentent un état anormal, ou si au moins un nombre donné de sous-ensemble (par exemple, au moins deux sous-ensembles sur trois) présentent un état anormal. En milieu naturel, il n'y a pas génération d'anomalie quand il y a détection sur un seul capteur uniquement, même après lissage temporel (prise en compte des pas de temps précédents pour fusionner les potentielles anomalies survenues peu avant).

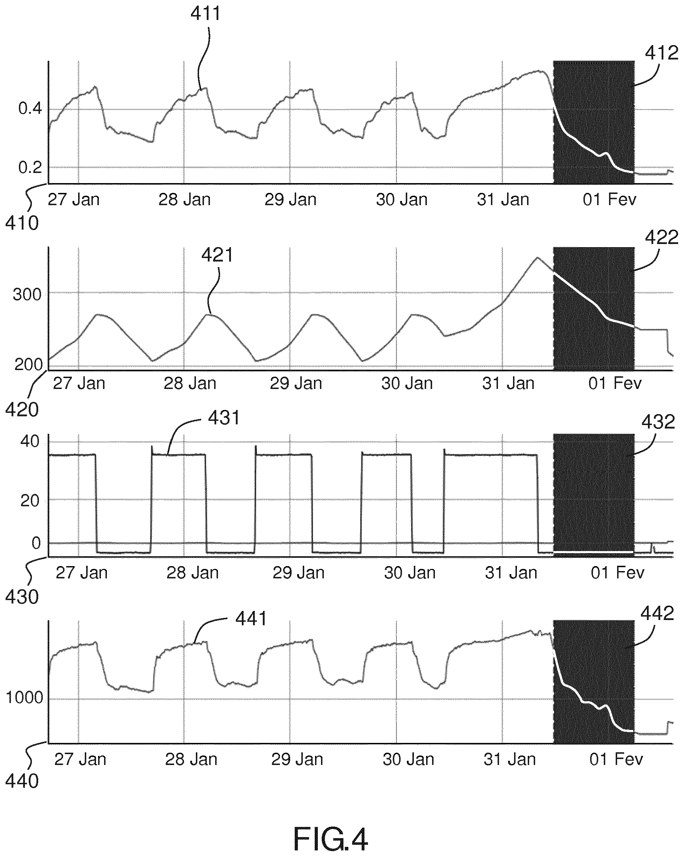

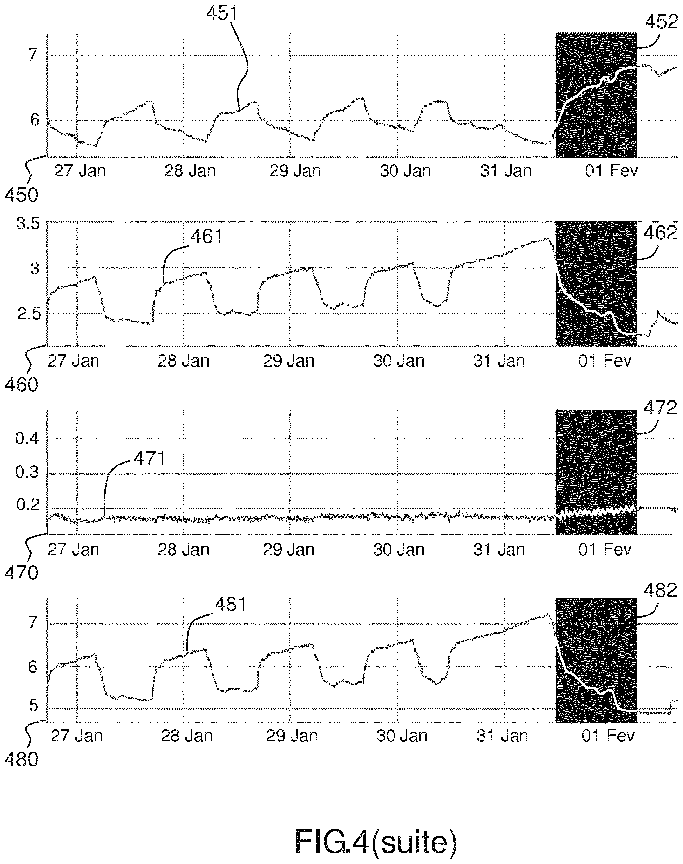

La figure 4 représente un exemple de détection d'une fenêtre temporelle anormale, dans un réseau de distribution d'eau potable.

Les courbes 410, 420, 430, 440, 450, 460, 470 et 480 représentent respectivement les évolutions de mesures de 8 capteurs de concentration en chlore 411, niveau d'un réservoir d'eau 421, débit 431, conductivité 441, température 451, TOC 461, turbidité 471 et UV254 481.

Ces mesures peuvent être obtenues à partir de différents capteurs, par exemple les capteurs 211, 212.

Les mesures sont ici effectuées sur une semaine, entre le 26 janvier et le 1^{er} février. Les mesures sont transformées par fenêtres glissantes de 24h, afin de détecter des fenêtres anormales. Dans cet exemple, les mesures suivent une distribution régulière jusqu'au 1^{e} février. Cette distribution régulière représente, jour après jour, le mélange de deux sources d'eau pour le remplissage d'un réservoir. Ensuite un problème dans le remplissage du réservoir entraine de une anomalie de qualité visible sur de nombreux paramètres sur la même fenêtre temporelle : la concentration en chlore diminue brusquement 412, le niveau d'eau dans le réservoir monte puis diminue également 422, le débit d'eau reste nul durant 24 h 432, la conductivité de l'eau s'effondre 442, la température augmente de manière importante 452, la TOC (concentration Totale en Carbone Organique) diminue 462, la turbidité augmente 472, l'absorbance des UV254 diminue 482.

Les différentes transformations de ces mesures sur la fenêtre temporelle permettent de mettre en évidence que les mesures sur cette fenêtre ne suivent pas leur schéma habituel, et ainsi de détecter une fenêtre temporelle anormale, composée de plusieurs pas de temps anormaux.

Ces courbes sont données à titre d'exemple uniquement, et l'homme de l'art pourra selon les types d'anomalies recherchées et/ou les capteurs disponibles, détecter des fenêtres temporelles anormales sur un autre ensemble de grandeurs physiques.

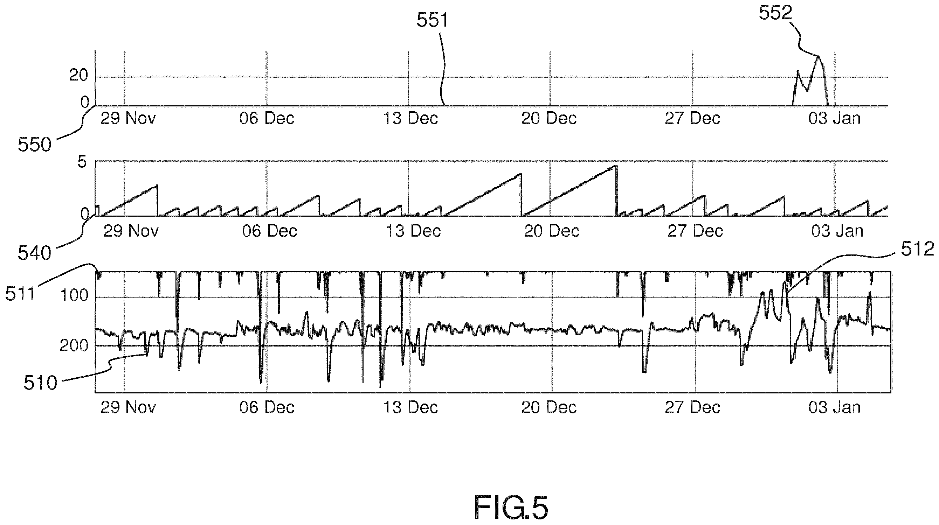

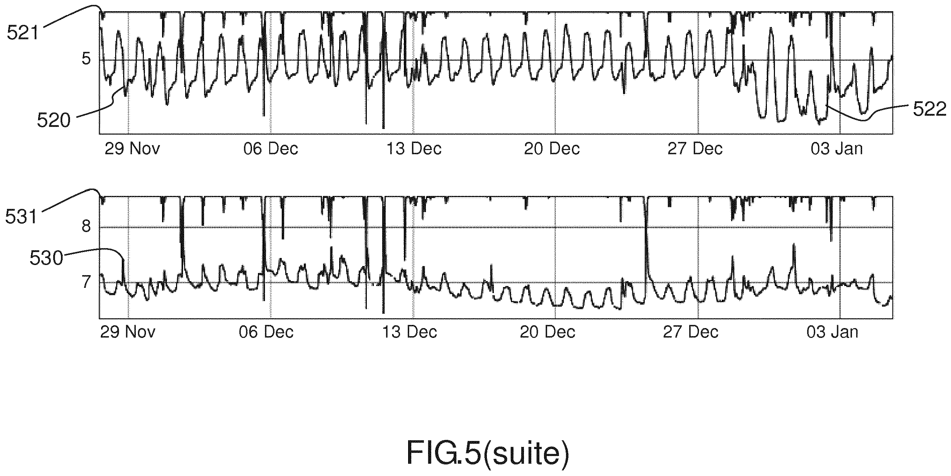

La figure 5 représente un exemple de détection d'une fenêtre temporelle anormale, dans de l'eau en milieu naturel.

La figure 5 représente des mesures issues d'un ensemble de capteurs d'une même sonde comprenant :
- des mesures de conductivité 510 ;
- des mesures d'un taux de dissolution d'oxygène 520 ;
- des mesures de pH 530.

Des données conjoncturelles (en l'occurrence un nombre de jours consécutifs sans pluie 540, récupéré à partir de données météorologiques) sont également utilisés.

Les mesures sont transformées afin d'obtenir des valeurs transformées 511, 521, 531 en prenant la médiane sur des périodes de 6h de la conductivité, du taux d'oxygène dissous et du pH.

La détection d'anomalies s'effectue par fenêtre temporelles de 6h: une fenêtre temporelle est analysée toutes les 6h. Un détecteur d'anomalies est appliqué à chaque fenêtre temporelle. Dans cet exemple, une variation est en premier lieu détectée sur chaque grandeur physique, et un état anormal de la fenêtre est détecté, si au moins deux grandeurs physiques présentent une variation à moins de 4 fenêtres temporelles d'écart. Ceci permet d'éviter de détecter une fenêtre anormale, si un capteur est défectueux.

Les mesures sont ici effectuées du 27 novembre au 5 janvier. Les mesures sont relativement régulières en novembre et décembre, en particulier en retirant les pas de temps correspondant à de la pluie (courbes bleues inversées) ; début janvier, survient une variation des paramètres pouvant refléter une pollution par rejet de produit dans le milieu, et se traduisant par une augmentation 512 de la conductivité, et une diminution 522 du taux d'oxygène dissous. Ces anomalies se traduisent par des variations anormalement élevées, qui permettent de détecter une anomalie de chacune de ces grandeurs physiques sur une fenêtre temporelle.

La courbe 550 représente une évaluation de l'intensité des anomalies sur chaque fenêtre temporelle de 6h. Dans cet exemple, l'intensité des anomalies est calculée, uniquement dans le cas où une anomalie est préalablement détectée, en retranchant la médiane cumulée des mesures sur l'historique de données disponibles de chaque grandeur physique aux valeurs transformées préalablement calculées, multipliées par 100 et divisées par cette même médiane cumulée pour normaliser ces écarts. Le maximum sur les différentes grandeurs physiques est alors calculé afin de produire l'indicateur représenté sur le graphe. Une valeur de 0 correspond donc à l'absence d'anomalies. Ceci n'est cependant donné qu'à titre d'exemple uniquement, et d'autre manières d'évaluer une criticité d'une anomalie se traduisant par des écarts importants entre les valeurs au sein d'une même fenêtre temporelle, ou par rapport à un historique de données, pourraient être utilisées. Il peut être observé que le détecteur d'anomalies ne détecte aucune fenêtre anormale en novembre et décembre, puis détecte une proportion importante de fenêtres anormales début janvier.

Ces courbes sont données à titre d'exemple uniquement, et l'homme de l'art pourra selon les types d'anomalies recherchées et/ou les capteurs disponibles, détecter des fenêtres temporelles anormales sur un autre ensemble de grandeurs physiques.

Revenant à la figure 2, Le processeur 240 est de plus configuré pour détecter 244 une anomalie dans le continuum d'eau, en fonction d'un nombre de fenêtres temporelles présentant un état anormal.

Dans un ensemble de modes de réalisation de l'invention, une anomalie est détectée dans le continuum d'eau, dès qu'une fenêtre temporelle présente un état anormal.

Dans un ensemble de modes de réalisation de l'invention, afin de limiter le nombre de fausses alertes si une seule fenêtre temporelle est identifiée comme anormale, une anomalie dans le continuum d'eau n'est détectée que si plusieurs fenêtres temporelles présentent un état anormal. Par exemple, une anomalie peut être détectée si un nombre prédéfini de fenêtres successives (par exemple 2, 3, 4, 10...) présente un état anormal. Une autre option consiste à détecter une anomalie, si, sur un nombre de fenêtres temporelles successives, un pourcentage de fenêtres successives supérieur à un seuil donné présente un état anormal. Par exemple, une anomalie peut être détectée dans le continuum d'eau si, parmi 10 fenêtres temporelles successives, au moins 75% présentent un état anormal. Ceci permet de ne générer une alerte que si un état anormal est avéré. Afin d'éviter qu'une alerte ne soit levée trop tard, les fenêtres temporelles utilisées peuvent êtres des fenêtres glissantes. Par exemple, des fenêtres de 6h de mesures peuvent êtres générées toutes les heures (ainsi, une fenêtre temporelle comprend 5h de mesures en commune avec la fenêtre précédente). Ceci permet donc en même temps de ne détecter une anomalie, que si celle-ci est avérée car mise en évidence sur plusieurs fenêtres temporelles, d'utiliser des fenêtres de temps longues, et d'éviter un retard trop important dans la détection d'anomalies.

Le processeur 240 est également configuré pour exécuter 245 sur les mesures un ensemble de règles prédéfinies de détection d'une variation d'une au moins des grandeurs physiques, et si une anomalie est détectée, affecter 246 à l'anomalie un type d'anomalies associé à une variation d'un sous-ensemble des grandeurs physiques, si la variation de l'une au moins des grandeurs du sous-ensemble est détectée.

Ces règles permettent de caractériser d'éventuelles anomalies dans le continuum d'eau. Il est possible d'utiliser des règles prédéterminées correspondant à des types d'anomalies connus. Par exemple, il est connu de l'homme de l'art qu'un changement de source d'eau se traduit par une modification de la conductivité, du pH et de la température de l'eau. Il est donc possible, en parallèle de la détection d'anomalie, d'exécuter des règles prédéfinies de caractérisation d'anomalie : ainsi, si une anomalie a été détectée, et qu'en parallèle une modification de la conductivité, du pH et de la température de l'eau a été observée, il peut être déduit que cette anomalie est un changement de source d'eau.

La combinaison de la détection est de la caractérisation d'anomalies est particulièrement efficace. En effet :
- la détection s'effectue en comparant des valeurs transformées de mesures à des valeurs transformées issues de mesures précédentes issues des mêmes capteurs sur un même continuum d'eau, ce qui permet de d'obtenir une détection de situation anormale particulièrement adaptée au continuum d'eau, sur la base d'occurrences statistiques. De plus, comme indiqué ci-dessus, une telle détection peut être mise en place rapidement avec des données d'entraînement limitées ;
- la caractérisation peut se baser sur des règles générales, puisqu'un même type d'anomalie se traduit, sur tous les continuums d'eau, par une variation des mêmes grandeurs physiques. Ainsi, la caractérisation ou le typage d'anomalies peut s'effectuer à partir de règles prédéfinies applicables à tous les systèmes aquatiques, sans avoir besoin d'avoir été paramétrées pour un continuum d'eau, ou des capteurs en particulier.

Ainsi, le dispositif selon l'invention permet de détecter des anomalies dans un continuum d'eau de manière fiable et adaptée aux caractéristiques de l'environnement étudié, et de déterminer leur type, avec sans avoir besoin d'avoir déjà observé chacune des anomalies possibles dans le continuum d'eau. Le dispositif selon l'invention peut donc être opérationnel très rapidement, avec peu d'observations préalables issues des capteurs 211, 212. Lorsqu'un ensemble de données d'entraînement plus important est disponible, et qu'un nombre suffisant d'anomalies ont été labellisées, le détecteur d'anomalies peut être remplacé, si la détection s'en trouve améliorée, par un détecteur d'anomalies détectant directement le type d'anomalie.

Selon différents modes de réalisation de l'invention, les règles de détection de variations des grandeurs physiques peuvent s'effectuer en parallèle de la détection des anomalies, ou bien uniquement si une anomalie a été détectée.

La détection de variations de grandeurs physiques peut par exemple s'effectuer en calculant une intensité de variation des grandeurs physiques, puis en comparant cette intensité à un seuil, la variation étant détectée si l'intensité de variation est supérieure au seuil. Cette intensité de variation peut être calculée de différentes manières. Par exemple, les intensités de variation peuvent être calculées à l'aide des valeurs transformées précédemment calculées pour une grandeur physique, par exemple une intensité de variation lente peut avoir déjà été calculée à partir d'un test de pente et du calcul de la pente, et l'on peut calculer une intensité de variation rapide en appliquant un quantité à 95% mobile à des valeurs représentatives d'une variation rapide, telles que des écarts de chaque mesure à une médiane sur une fenêtre temporelle. Ainsi, la moyenne des valeurs absolues des valeurs représentatives d'une variation rapide peut être calculée. Si cette moyenne est supérieure à un seuil, correspondant ici à la limite entre les 95% les plus bas, et les 5% les plus élevés des moyenne des valeurs absolues des valeurs représentatives d'une variation rapide sur un ensemble de fenêtres temporelles précédentes issues de données historiques, une variation de la grandeur physique est détectée. La moyenne peut être remplacée ici par d'autres valeurs caractéristiques, comme par exemple la somme des valeurs absolues des valeurs représentatives d'une variation rapide. Le seuil peut être calculé à partir des données historiques en ajoutant à la médiane 3 écart-types. Cela représente alors une valeur haute de la distribution des données. Cependant, ces règles sont données à titre d'exemple uniquement, et l'homme de l'art pourra mettre en oeuvre tout type de règle adaptée permettant de détecter une variation particulièrement élevée d'une grandeur physique, utilisant ou non les valeurs transformées calculées par l'au moins une transformation 242.

Selon différents modes de réalisation de l'invention, l'intensité de variations peut être calculée pour tout ou partie des grandeurs physiques.

Pour certaines grandeurs physiques, une variation peut être considérée comme significative ou problématique, seulement pour un sens de variation donné. Par exemple, une diminution de la concentration en chlore pourrait être considérée comme problématique, alors qu'une augmentation ne le serait pas. Dans un ensemble de modes de réalisation de l'invention, une variation de certaines grandeurs physiques n'est retenue que si le sens de variation de la grandeur physique sur les mesures est conforme à un sens de variation.

Dans un ensemble de modes de réalisation de l'invention, les intensités de variations des grandeurs physiques permettent également de déterminer une criticité de l'anomalie. Un score de criticité peut ainsi être affecté à l'anomalie en fonction des intensités des variations des grandeurs physiques du sous-ensemble associé à l'anomalie. Ce score est calculé selon les intensités des différents paramètres calculées préalablement. Ces intensités sont comparées aux intensités calculées sur les fenêtres temporelles précédentes dans l'historique de données, uniquement pour les paramètres critiques. Elles sont alors normalisées par rapport au maximum robuste observé sur chaque paramètre, puis le maximum est pris, afin de déterminer la criticité comme un nombre entre 0 et 1.

L'homme du métier peut prévoir de nombreuses règles d'affectation de type à une anomalie, en fonction des capteurs disponibles, et des grandeurs physiques associées aux types d'anomalies recherchées.

Par exemple, le tableau suivant fournit quelques exemples de types d'anomalies pouvant être affectés, en fonction des variations anormales de grandeurs physiques :

| **Grandeurs ayant une variation** | **Types d'anomalies** |
|---|---|
| Concentration en chlore, température, TOC, UV254, nombre de bactéries | Croissance Bactérienne |
| Conductivité, pH, température | Mélange d'eau |
| Concentration en chlore, pH, couleur, turbidité, UV254 | Eaux colorées |
| Turbidité, particules | Mouvements de sédiments |
| Toutes. | Anomalie globale |

Ces possibilités sont données à titre d'exemple uniquement, et l'homme de l'art peut choisir, pour chaque anomalie, les grandeurs physiques dont la variation permet de mieux la caractériser. L'invention permet ainsi de caractériser un nombre important d'anomalies différentes.

Selon différents modes de réalisation de l'invention, un type d'anomalie peut être détecté, si tout ou parties des grandeurs physiques associées varient. Dans l'exemple du tableau ci-dessus, une anomalie pourrait être caractérisée avec le type « mélange d'eau », soit si les trois grandeurs conductivité, pH, température ont une variation anormale, soit si deux des trois au moins varient.

Une fois une anomalie détectée, celle-ci peut, dans un ensemble de modes de réalisation de l'invention, être affichée à un opérateur via l'interface 250. L'interface 250 peut notamment afficher le type d'anomalie, le lieu où cette anomalie a été détectée et, le cas échéant, la criticité de l'anomalie.

Les anomalies détectées peuvent être croisées avec d'autres sources d'informations, telles que des plaintes client, afin de confirmer ou d'infirmer l'anomalie.

L'opérateur peut ainsi prendre toutes les mesures nécessitées par l'anomalie. Dans un ensemble de modes de réalisation de l'invention, l'opérateur peut également valider ou non l'anomalie, c'est-à-dire indiquer, après vérification, si la détection de l'anomalie était justifiée ou non. Ceci permet, lorsque le détecteur d'anomalies est une machine d'apprentissage automatique, de disposer de données d'entrainement plus fiables, et donc d'améliorer les performances de la détection d'anomalies.

Dans un ensemble de modes de réalisation de l'invention, une anomalie détectée peut générer un événement. Différents événements peuvent être générés, en fonction des mesures issues de différentes sections d'un réseau de distribution d'eau, ou différents capteurs. Les événements peuvent également être combinés entre eux, une combinaison d'événements locaux d'un réseau de distribution d'eau pouvant être interprétée comme un événement global. Si des événements similaires sont détectés sur plusieurs capteurs, la zone couverte peut être affichée à l'utilisateur pour qu'il prenne les mesures adéquates la concernant. Si des phénomènes sont détectés à la fois en sortie d'usine et sur le reste du réseau, il s'agit est possible de déduire que l'anomalie concerne l'usine de traitement de l'eau, et un événement adéquat peut être généré. Il est donc nécessaire d'enquêter à ce niveau et pas de celui du réseau.

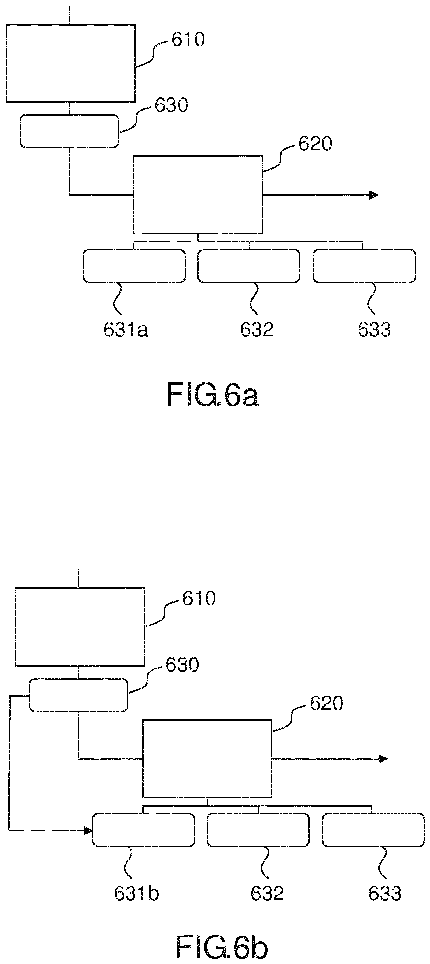

Les figures 6a et 6b représentent deux exemples de combinaisons d'événements sur un réseau de distribution d'eau, dans un ensemble de modes de mise en oeuvre de l'invention.

Dans ces deux exemples, un réseau de distribution d'eau potable 620 est situé en aval d'une usine de traitement d'eau 610. Plusieurs générateurs d'événements sont basés sur des détections d'anomalies telles que représentées dans les exemples relatifs à la figure 2. Un premier générateur d'événement 630 détecte d'éventuelles anomalies à partir de mesures effectuées en sortie de l'usine de production d'eau potable.

Dans l'exemple de la figure 6a, trois générateurs d'événements 631a, 632, 633 sont exécutés en parallèle, à partir de mesures de grandeurs physiques en trois points différents du réseau. Les événements ainsi combinés peuvent être combinés en événements du réseau dans son ensemble. Par exemple, un mélange d'eau devrait être visible sur plusieurs points d'une même zone.

Dans l'exemple de la figure 6b, le détecteur d'événement 631a a été modifié en un détecteur d'événement 631b, prenant en compte la survenue d'événements 630 en sortie de l'usine de production d'eau potable. Ainsi, lorsqu'une anomalie est détectée par les mesures utilisées par le détecteur d'événement 631b, la caractérisation du type d'anomalie ou d'événement dépend de la détection d'événement 630. Par exemple, un changement dans la production devrait pouvoir ne pas être généré sur les capteurs du réseau parce que déjà détecté en sortie d'usine.

Ces exemples démontrent la capacité de l'invention à détecter des anomalies ou événements dans un réseau de distribution d'eau, tout en prenant en compte les interactions entre les différents points du réseau, afin de détecter des anomalies plus globales.

Cependant, l'invention n'est pas limitée à ces exemples, et une combinaison d'événements telle que décrite en référence aux figures 6a et 6b pourrait par exemple être appliquée à la détection d'anomalies en milieu naturel.

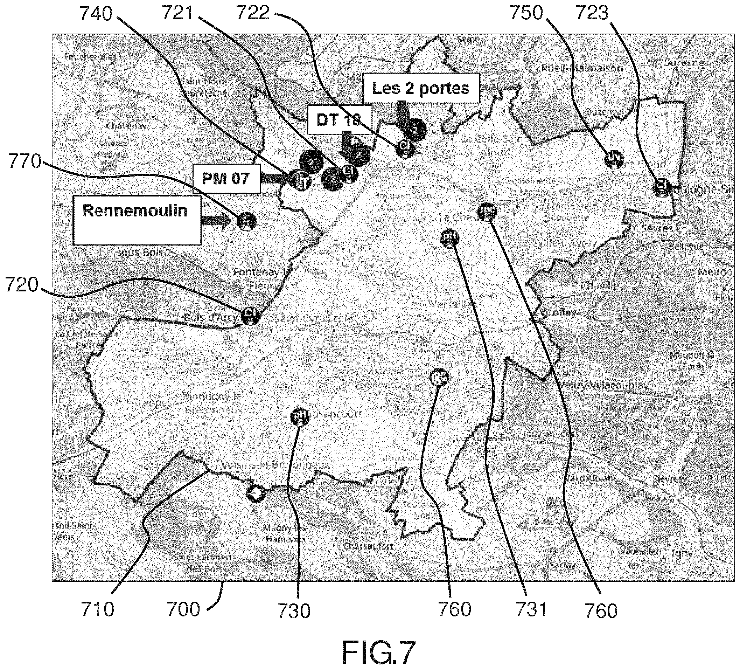

La figure 7 représente une interface de présentation d'événements survenant dans un réseau de distribution d'eau à un opérateur.

L'interface 700 est configurée pour présenter les anomalies détectées par un dispositif selon l'invention dans un réseau de distribution d'eau à un opérateur. Dans cet exemple, les anomalies sont détectées sur un secteur 710, et sont localisées sur une carte. Les types d'anomalies sont représentés par différents pictogrammes. Dans cet exemple, les pictogrammes représentent respectivement les positions des points de mesure : concentration en chlore 720, 721, 722 et 723, du pH 730 et 731, de la température 740, de l'absorbance de l'eau aux UV 750, de la TOC 760, de sondes multi-paramètres 760 et de points de prélèvement ponctuels 770.

Cet exemple démontre ainsi la capacité de l'invention à montrer de manière claire intuitive à un opérateur les différentes anomalies survenant dans un réseau de distribution d'eau. Cependant l'invention n'est pas restreinte à cet exemple, et d'autres types de représentation pourraient être utilisés. De la même manière, cette représentation pourrait être utilisée pour de l'eau en milieu naturel.

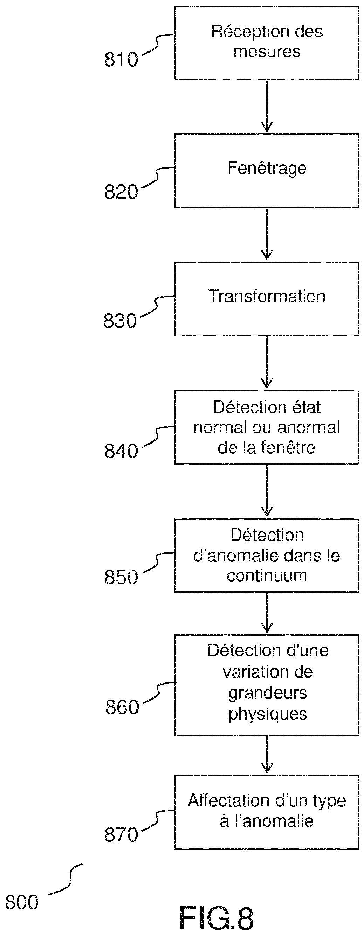

La figure 8 représente une méthode 800 de détection et de caractérisation d'anomalies dans un continuum d'eau selon l'invention.

La méthode 800 comprend la réception 810 de mesures d'une pluralité de grandeurs physiques issues d'une pluralité de capteurs de la pluralité de grandeurs physiques dans le continuum d'eau.

La méthode 800 comprend de plus la génération 820 d'une pluralité de fenêtres temporelles de mesures.

La méthode 800 comprend de plus, pour chaque fenêtre de ladite pluralité :
- l'obtention d'un ensemble de valeurs transformées par au moins une transformation 830 des mesures sur la fenêtre temporelle ;
- la détection 840 d'un un état normal ou anormal de la fenêtre temporelle, ladite détection étant paramétrée à partir d'ensembles de valeurs transformées issues de l'application de l'au moins une transformation à des fenêtres temporelles de mesures précédentes de l'au moins un capteur.

Dans une ensemble de modes de réalisations de l'invention, une au moins des transformations 830 est une transformation des mesures en valeurs représentatives de la variabilité des mesures au sein de la fenêtre temporelle.

Dans un ensemble de modes de réalisation de l'invention, la transformation des mesures en valeurs représentatives de la variabilité des mesures au sein de la fenêtre temporelle est réalisée en soustrayant à chaque mesure sur la fenêtre temporelle une médiane desdites mesures sur une fenêtre temporelle glissante.

Dans un ensemble de modes de réalisation de l'invention, la transformation ne conserve que des mesures correspondant à un sens de variation prédéfini.

Dans un ensemble de modes de réalisations de l'invention, une au moins des transformations 830 consiste en un test de pente sur la fenêtre temporelle.

Dans un ensemble de modes de réalisation de l'invention, le test de pente est un test de tendance de Mann-Kendall.

Dans un ensemble de modes de réalisation de l'invention, au moins deux des capteurs sont des capteurs d'une même grandeur physique en deux points du continuum d'eau, et une des transformations des mesures sur une fenêtre temporelle comprend une différence temporelle entre les mesures des deux capteurs.

Dans un ensemble de modes de réalisation de l'invention, l'ensemble de valeurs transformées est un vecteur, et le détecteur d'anomalies est une Machine à Vecteurs de Support à une classe.

Dans un ensemble de modes de réalisation de l'invention, l'ensemble de valeurs transformées est obtenu par au moins deux transformations des mesures sur la fenêtre temporelle, et la détection d'anomalies détermine un état normal ou anormal de la fenêtre temporelle à partir de l'ensemble des valeurs transformées.

Dans un ensemble de modes de réalisation de l'invention, l'ensemble de valeurs transformées est obtenu par au moins deux transformations des mesures sur la fenêtre temporelle, et la détection d'anomalies :
- détecte des états normaux ou anormaux d'au moins deux sous-ensembles de valeurs transformées respectivement par lesdites au moins deux transformations des mesures sur la fenêtre temporelle ;
- détecte l'état normal ou anormal de la fenêtre temporelle à partir d'une combinaison desdits états normaux ou anormaux des sous-ensembles.

La méthode 800 comprend de plus la détection 850 d'une anomalie dans le continuum d'eau, en fonction d'un nombre de fenêtres temporelles présentant un état anormal.

La méthode 800 comprend de plus l'exécution 860 d'un ensemble de règles prédéfinies de détection d'une variation d'une au moins des grandeurs physiques.

Dans un ensemble de modes de réalisation de l'invention, la méthode 800 comprend un calcul d'une intensité de variation des grandeurs physiques, et l'ensemble de règles prédéfinies de caractérisation comprend une règle prédéfinie de détection d'une variation de d'une grandeur physique, si l'intensité de variation de ladite grandeur physique sur les mesures est supérieure à un seuil de variation normale.

Dans un ensemble de modes de réalisation de l'invention, la détection de la variation de la grandeur physique ne détecte une variation que si la variation de la grandeur physique est conforme à un sens de variation.

La méthode 800 comprend enfin, si une anomalie est détectée, l'affectation 870 à l'anomalie d'un type d'anomalies associé à une variation d'un sous-ensemble des grandeurs physiques, si la variation de l'une au moins des grandeurs du sous-ensemble est détectée.

Dans un ensemble de modes de réalisation de l'invention, la méthode 800 comprend l'affectation d'un indicateur de criticité à l'anomalie, en fonction des intensités des variations des grandeurs physiques du sous-ensemble.

Dans un ensemble de modes de réalisation de l'invention, la méthode 800 comprend, si une anomalie est détectée, l'affectation à l'anomalie de l'un au moins des types suivants :
- un type « croissance bactérienne », en cas de variation de grandeurs physiques d'un sous-ensemble comprenant : une diminution de la concentration en chlore, une augmentation de température, une augmentation de la teneur en carbone organique total, une augmentation de l'absorbance de lumière Ultraviolette de longueur d'onde 254 nm, une augmentation du nombre de bactéries ;
- un type « mélange d'eau », en cas de variations de grandeurs physiques d'un sous-ensemble comprenant une conductivité, un pH ; une température ;
- un type « eaux colorées », en cas de variations de grandeurs physiques d'un sous-ensemble comprenant une concentration en chlore, un pH, une augmentation de couleur, une augmentation de turbidité, une augmentation de l'absorbance de lumière Ultraviolette de longueur d'onde 254 nm ;
- une survitesse, en cas d'augmentation anormale de grandeurs physiques parmi un sous-ensemble comprenant la turbidité et les particules.

Dans un ensemble de modes de réalisation de l'invention, la méthode 800 comprend, si la sortie des de règles prédéfinies de détection d'une variation d'une au moins des grandeurs physiques ne permet pas l'affectation d'un type à une anomalie, l'affectation à cette anomalie d'un type inconnu.

Dans un ensemble de modes de réalisation de l'invention, la méthode 800 comprend une étape d'utilisation d'une interface pour afficher l'anomalie et son type à un opérateur.

Dans un ensemble de modes de réalisation de l'invention, méthode 800 comprend :
- l'utilisation de l'interface pour recevoir de l'opérateur un label concernant l'anomalie ;
- l'ajout de la fenêtre temporelle de valeurs, et du label concernant l'anomalie aux données d'entraînement.

La méthode 800 permet ainsi de détecter différents types d'anomalies dans un continuum d'eau, et de leur affecter des types. Tous les modes de réalisation décrits en référence aux figures 2 à 7 sont applicables à la méthode 800.

Les exemples ci-dessus démontrent la capacité de l'invention à détecter des événements ou anomalies liés à la qualité de l'eau. Ils ne sont cependant donnés qu'à titre d'exemple et ne limitent en aucun cas la portée de l'invention, définie dans les revendications ci-dessous.

## Revendications

1. Dispositif (100, 200) apte à détecter et caractériser des d'anomalies dans un continuum d'eau, comprenant :
- au moins un lien de communication (221, 222), respectivement vers au moins un capteur (211, 212) d'au moins une grandeur physique dans le continuum d'eau ;
- un processeur (240) configuré pour :
- recevoir des mesures de l'au moins un capteur par l'au moins un lien de communication ;
- générer une pluralité de fenêtres temporelles des mesures ;
- pour chaque fenêtre temporelle de ladite pluralité :
- obtenir un ensemble de valeurs transformées par au moins une transformation (242) des mesures sur la fenêtre temporelle ;
- appliquer un détecteur d'anomalies (243) à l'ensemble de valeurs transformées afin de détecter un état normal ou anormal de la fenêtre temporelle, ledit détecteur d'anomalies étant paramétré à partir d'ensembles de valeurs transformées issues de l'application de l'au moins une transformation à des fenêtres temporelles de mesures précédentes de l'au moins un capteur ;
- détecter (244) une anomalie dans le continuum d'eau, en fonction d'un nombre de fenêtres temporelles présentant un état anormal ;
- exécuter (245) sur les mesures un ensemble de règles prédéfinies de détection d'une variation d'une au moins des grandeurs physiques ;
- si une anomalie dans le continuum d'eau est détectée, affecter (246) à l'anomalie un type d'anomalies associé à une variation d'un sous-ensemble prédéfini des grandeurs physiques, si la variation de l'une au moins des grandeurs du sous-ensemble est détectée.

2. Dispositif selon la revendication 1, dans lequel une des transformations des mesures sur une fenêtre temporelle est une transformation des mesures en valeurs représentatives de la variabilité des mesures au sein de la fenêtre temporelle.

3. Dispositif selon la revendication 2, dans lequel la transformation des mesures en valeurs représentatives de la variabilité des mesures au sein de la fenêtre temporelle est réalisée en soustrayant à chaque mesure sur la fenêtre temporelle une médiane desdites mesures sur une fenêtre temporelle glissante comprenant au moins ladite fenêtre temporelle.

4. Dispositif selon l'une des revendications 1 à 3, dans lequel la transformation ne conserve que des mesures correspondant à un sens de variation prédéfini.

5. Dispositif selon la revendication 1, dans lequel une des transformations des mesures sur une fenêtre temporelle consiste en un test de pente sur la fenêtre temporelle.

6. Dispositif selon la revendication 1, dans lequel au moins deux des capteurs sont des capteurs d'une même grandeur physique en deux points du continuum d'eau, et une des transformations des mesures sur une fenêtre temporelle comprend une différence temporelle entre les mesures des deux capteurs.

7. Dispositif selon l'une des revendications 1 à 6, dans lequel l'ensemble de valeurs transformées est un vecteur, et le détecteur d'anomalies est une Machine à Vecteurs de Support à une classe.

8. Dispositif selon l'une des revendications 1 à 7, dans lequel l'ensemble de valeurs transformées est obtenu par au moins deux transformations des mesures sur la fenêtre temporelle, et le détecteur d'anomalies est configuré pour déterminer un état normal ou anormal de la fenêtre temporelle à partir de l'ensemble des valeurs transformées.

9. Dispositif selon l'une des revendications 1 à 7, dans lequel l'ensemble de valeurs transformées est obtenu par au moins deux transformations des mesures sur la fenêtre temporelle, et le détecteur d'anomalies est configuré pour :
- détecter des états normaux ou anormaux d'au moins deux sous-ensembles de valeurs transformées respectivement par lesdites au moins deux transformations des mesures sur la fenêtre temporelle ;
- détecter l'état normal ou anormal de la fenêtre temporelle à partir d'une combinaison desdits états normaux ou anormaux des sous-ensembles.

10. Dispositif selon l'une des revendications 1 à 9, dans lequel le processeur est configuré pour calculer une intensité de variation des grandeurs physiques, et l'ensemble de règles prédéfinies de caractérisation comprend une règle prédéfinie de détection d'une variation de d'une grandeur physique, si l'intensité de variation de ladite grandeur physique sur les mesures est supérieure à un seuil de variation normale.

11. Dispositif selon la revendication 10, dans lequel le processeur est configuré pour affecter un indicateur de criticité à l'anomalie, en fonction des intensités des variations des grandeurs physiques du sous-ensemble.

12. Dispositif selon l'une des revendications 1 à 11, dans lequel la détection de la variation de la grandeur physique ne détecte une variation que si la variation de la grandeur physique est conforme à un sens de variation.

13. Dispositif selon la revendication 1 à 12, dans lequel le processeur est configuré, si une anomalie est détectée, pour affecter à l'anomalie l'un au moins des types suivants :
- un type « croissance bactérienne », en cas de variation de grandeurs physiques d'un sous-ensemble comprenant : une diminution de la concentration en chlore, une augmentation de température, une augmentation de la teneur en carbone organique total, une augmentation de l'absorbance de lumière Ultraviolette de longueur d'onde 254 nm, une augmentation du nombre de bactéries ;
- un type « mélange d'eau », en cas de variations de grandeurs physiques d'un sous-ensemble comprenant une conductivité, un pH ; une température ;
- un type « eaux colorées », en cas de variations les grandeurs physiques d'un sous-ensemble comprenant une concentration en chlore, un pH, une augmentation de couleur, une augmentation de turbidité, une augmentation de l'absorbance de lumière Ultraviolette de longueur d'onde 254 nm ;
- une survitesse, en cas d'augmentation anormale de grandeurs physiques parmi un sous-ensemble comprenant la turbidité et les particules.

14. Dispositif selon l'une des revendications 1 à 13, dans lequel le processeur est configuré, si la sortie des de règles prédéfinies de détection d'une variation d'une au moins des grandeurs physiques ne permet pas l'affectation d'un type à une anomalie, pour affecter à cette anomalie un type inconnu.

15. Dispositif selon l'une des revendications 1 à 14, comprenant une interface (250) pour afficher l'anomalie et son type à un opérateur.

16. Dispositif selon la revendication 15, dans lequel :
- l'interface est configurée pour recevoir de l'opérateur un label concernant l'anomalie ;
- la fenêtre temporelle de valeurs, et le label concernant l'anomalie sont ajoutés aux données d'entraînement.

17. Méthode (800) de détection et de caractérisation d'anomalies dans un continuum d'eau, comprenant :
- la réception (810) de mesures d'une pluralité de grandeurs physiques issues d'une pluralité de capteurs de la pluralité de grandeurs physiques dans le continuum d'eau ;
- la génération (820) d'une pluralité de fenêtres temporelles de mesures ;
- pour chaque fenêtre de ladite pluralité :
- l'obtention d'un ensemble de valeurs transformées par au moins une transformation (830) des mesures sur la fenêtre temporelle ;
- la détection (840) d'un état normal ou anormal de la fenêtre temporelle, ladite détection étant paramétré à partir d'ensembles de valeurs transformées issues de l'application de l'au moins une transformation à des fenêtres temporelles de mesures précédentes de l'au moins un capteur ;
- la détection (850) d'une anomalie dans le continuum d'eau, en fonction d'un nombre de fenêtres temporelles présentant un état anormal ;
- l'exécution (860) d'un ensemble de règles prédéfinies de détection d'une variation d'une au moins des grandeurs physiques ;
- si une anomalie dans le continuum d'eau est détectée, l'affectation (870) à l'anomalie un type d'anomalies associé à une variation d'un sous-ensemble prédéfini des grandeurs physiques, si la variation de l'une au moins des grandeurs du sous-ensemble est détectée.

18. Produit programme d'ordinateur comprenant des instructions de code de programme enregistrés sur un support lisible par un ordinateur comprenant un processeur pour la détection d'anomalies dans un continuum d'eau, ledit programme d'ordinateur comprenant des moyens de programmation lisibles par ordinateur pour :
- recevoir des mesures d'une pluralité de grandeurs physiques issues d'une pluralité de capteurs de la pluralité de grandeurs physiques dans le continuum d'eau ;
- générer une pluralité de fenêtres temporelles de mesures ;
- pour chaque fenêtre de ladite pluralité :
- obtenir un ensemble de valeurs transformées par au moins une transformation des mesures sur la fenêtre temporelle ;
- appliquer un détecteur d'anomalies à l'ensemble de valeurs transformées afin de détecter un état normal ou anormal de la fenêtre temporelle, ledit détecteur d'anomalies étant paramétré à partir d'ensembles de valeurs transformées issues de l'application de l'au moins une transformation à des fenêtres temporelles de mesures précédentes de l'au moins un capteur ;
- détecter une anomalie dans le continuum d'eau, en fonction d'un nombre de fenêtres temporelles présentant un état anormal ;
- exécuter un ensemble de règles prédéfinies de détection d'une variation d'une au moins des grandeurs physiques ;
- si une anomalie dans le continuum d'eau est détectée, affecter à l'anomalie un type d'anomalies associé à une variation d'un sous-ensemble prédéfini des grandeurs physiques, si la variation de l'une au moins des grandeurs du sous-ensemble est détectée.

## Patentansprüche

1. Vorrichtung (100, 200), die geeignet ist, Anomalien in einem Wasserkontinuum zu erfassen und zu charakterisieren, umfassend:
- mindestens eine Kommunikationsverbindung (221, 222) zu mindestens eine Sensor (211, 212) für mindestens eine physikalische Größe in dem Wasserkontinuum;
- einen Prozessor (240), der konfiguriert ist zum:
- Empfangen von Messungen von dem mindestens einen Sensor über die mindestens eine Kommunikationsverbindung;
- Erzeugen einer Vielzahl von Zeitfenstern der Messungen;
- für jedes Zeitfenster der Vielzahl:
- Erzielen eines Satzes von Werten, die durch mindestens eine Transformation (242) der Messungen über das Zeitfenster transformiert wurden;
- Anwenden eines Anomaliedetektors (243) auf den Satz transformierter Werte, um einen normalen oder abnormalen Zustand des Zeitfensters zu erfassen, wobei der Anomaliedetektor anhand von Sätzen transformierter Werte parametrisiert wird, die aus der Anwendung der mindestens einen Transformation auf Zeitfenster vorhergehender Messungen des mindestens einen Sensors stammen;
- Erfassen (244) einer Anomalie im Wasserkontinuum, entsprechend einer Anzahl von Zeitfenstern, die einen abnormalen Zustand aufweist;
- Ausführen (245) eines Satzes vordefinierter Regeln zum Erfassen einer Variation mindestens einer der physikalischen Größen an den Messungen;
- wenn eine Anomalie im Wasserkontinuum erfasst wird, Zuweisen (246) zu der Anomalie eines Typs von Anomalien, der mit einer Variation einer vordefinierten Teilmenge der physikalischen Größen verbunden ist, wenn die Variation mindestens einer der Größen der Teilmenge erfasst wird.

2. Vorrichtung nach Anspruch 1, wobei eine der Transformationen der Messungen über ein Zeitfenster eine Transformation der Messungen in Werte ist, die für die Variabilität der Messungen innerhalb des Zeitfensters repräsentativ sind.

3. Vorrichtung nach Anspruch 2, wobei die Transformation der Messungen in Werte, die für die Variabilität der Messungen innerhalb des Zeitfensters repräsentativ sind, dadurch erfolgt, dass von jeder Messung über das Zeitfenster ein Median der Messungen über ein gleitendes Zeitfenster, das mindestens das Zeitfenster umfasst, subtrahiert wird.

4. Vorrichtung nach einem der Ansprüche 1 bis 3, wobei die Transformation nur Messungen beibehält, die einer vordefinierten Variationsrichtung entsprechen.

5. Vorrichtung nach Anspruch 1, wobei eine der Transformationen der Messungen über ein Zeitfenster aus einem Steigungstest über das Zeitfenster besteht.

6. Vorrichtung nach Anspruch 1, wobei mindestens zwei der Sensoren Sensoren für eine gleiche physikalische Größe an zwei Punkten des Wasserkontinuums sind und eine der Transformationen der Messungen über ein Zeitfenster eine zeitliche Differenz zwischen den Messungen der beiden Sensoren umfasst.

7. Vorrichtung nach einem der Ansprüche 1 bis 6, wobei der Satz transformierter Werte ein Vektor ist und der Anomaliedetektor eine Einklassen-Support-Vektor-Maschine ist.

8. Vorrichtung nach einem der Ansprüche 1 bis 7, wobei der Satz transformierter Werte durch mindestens zwei Transformationen der Messungen über das Zeitfenster erzielt wird, und der Anomaliedetektor so konfiguriert ist, dass er anhand des Satzes transformierter Werte einen normalen oder abnormalen Zustand des Zeitfensters bestimmt.

9. Vorrichtung nach einem der Ansprüche 1 bis 7, wobei der Satz transformierter Werte durch mindestens zwei Transformationen der Messungen über das Zeitfenster erzielt wird, und der Anomaliedetektor konfiguriert ist zum;
- Erfassen von normalen oder abnormalen Zuständen von mindestens zwei Teilmengen von Werten, die jeweils durch die mindestens zwei Transformationen der Messungen über das Zeitfenster transformiert wurden;
- Erfassen des normalen oder abnormalen Zustandes des Zeitfensters anhand einer Kombination der normalen oder abnormalen Zustände der Teilmengen.

10. Vorrichtung nach einem der Ansprüche 1 bis 9, wobei der Prozessor so konfiguriert ist, dass er eine Variationsintensität der physikalischen Größen berechnet, und der Satz vordefinierter Charakterisierungsregeln eine vordefinierte Regel zur Erfassung einer Variation einer physikalischen Größe umfasst, wenn die Variationsintensität der physikalischen Größe über die Messungen größer als ein Schwellenwert für normale Variationen ist.

11. Vorrichtung nach Anspruch 10, wobei der Prozessor so konfiguriert ist, dass er der Anomalie einen Kritizitätsindikator entsprechend den Intensitäten der Variationen der physikalischen Größen der Teilmenge zuordnet.

12. Vorrichtung nach einem der Ansprüche 1 bis 11, wobei die Erfassung der Variation der physikalischen Größe eine Variation nur dann erfasst, wenn die Variation der physikalischen Größe mit einer Variationsrichtung übereinstimmt.

13. Vorrichtung nach Anspruch 1 bis 12, wobei der Prozessor so konfiguriert ist, dass er, wenn eine Anomalie festgestellt wird, der Anomalie mindestens einen der folgenden Typen zuordnet:
- einen Typ "Bakterienwachstum", im Fall der Variation physikalischer Größen einer Teilmenge, die Folgendes umfasst: eine Abnahme der Chlorkonzentration, eine Temperaturerhöhung, eine Zunahme des Gesamtgehalts an organischem Kohlenstoff, eine Zunahme der Extinktion von ultraviolettem Licht der Wellenlänge 254 nm, eine Zunahme der Anzahl von Bakterien;
- einen Typ "Mischwasser", im Fall von Variationen physikalischer Größen einer Teilmenge, die eine Leitfähigkeit, einen pH-Wert, eine Temperatur umfasst;
- einen Typ "gefärbtes Wasser", im Fall von Variationen die physikalischen Größen einer Teilmenge, die eine Chlorkonzentration, einen pH-Wert, eine Zunahme der Farbe, eine Zunahme der Trübung, eine Zunahme der Absorption von ultraviolettem Licht mit einer Wellenlänge von 254 nm umfasst;
- eine Übergeschwindigkeit, im Fall eines abnormalen Anstiegs physikalischer Größen aus einer Teilmenge, die Trübung und Partikel umfasst.

14. Vorrichtung nach einem der Ansprüche 1 bis 13, wobei der Prozessor so konfiguriert ist, dass er, wenn die Ausgabe der vordefinierten Regeln zur Erfassung einer Variation mindestens einer der physikalischen Größen die Zuweisung eines Typs zu einer Anomalie nicht zulässt, dieser Anomalie einen unbekannten Typ zuweist.

15. Vorrichtung nach einem der Ansprüche 1 bis 14, die eine Schnittstelle (250) zum Anzeigen der Anomalie und ihres Typs an einen Bediener umfasst.

16. Vorrichtung nach Anspruch 15, wobei:
- die Schnittstelle so konfiguriert ist, dass sie von dem Bediener ein Label bezüglich der Anomalie empfängt;
- das Wertezeitfenster und das Label bezüglich der Anomalie zu den Trainingsdaten hinzugefügt werden.

17. Verfahren (800) zum Erfassen und Charakterisieren von Anomalien in einem Wasserkontinuum, umfassend:
- Empfangen (810) von Messungen einer Vielzahl von physikalischen Größen, die von einer Vielzahl von Sensoren der Vielzahl von physikalischen Größen in dem Wasserkontinuum stammen;
- Erzeugen (820) einer Vielzahl von Zeitfenstern für Messungen;
- für jedes Fenster der Vielzahl:
- Erzielen eines Satzes von Werten, die durch mindestens eine Transformation (830) der Messungen über das Zeitfenster transformiert wurden;
- Erfassen (840) eines normalen oder abnormalen Zustands des Zeitfensters, wobei die Erfassung anhand von Sätzen transformierter Werte parametrisiert wird, die aus der Anwendung der mindestens einen Transformation auf Zeitfenster von vorherigen Messungen des mindestens einen Sensors stammen;
- Erfassen (850) einer Anomalie im Wasserkontinuum, entsprechend einer Anzahl von Zeitfenstern, die einen abnormalen Zustand aufweist;
- Ausführen (860) eines Satzes von vordefinierten Regeln zum Erfassen einer Variation von mindestens einer der physikalischen Größen;
- wenn eine Anomalie im Wasserkontinuum erkannt wird, Zuweisen (870) eines Typs von Anomalien, der mit einer Variation einer vordefinierten Teilmenge der physikalischen Größen verbunden ist, zu der Anomalie, wenn die Variation mindestens einer der Größen der Teilmenge erkannt wird.

18. Computerprogrammprodukt mit Programmcodeanweisungen, die auf einem computerlesbaren Medium aufgezeichnet sind, das einen Prozessor zur Erfassung von Anomalien in einem Wasserkontinuum umfasst, wobei das Computerprogramm computerlesbare Programmiermittel umfasst zum:
- Empfangen von Messungen einer Vielzahl von physikalischen Größen von einer Vielzahl von Sensoren der Vielzahl von physikalischen Größen in dem Wasserkontinuum;
- Erzeugen einer Vielzahl von Zeitfenstern von Messungen;
- für jedes Fenster der Vielzahl:
- Erzielen eines Satzes von Werten, die durch mindestens eine Transformation der Messungen über das Zeitfenster transformiert wurden;
- Anwenden eines Anomaliedetektors auf den Satz transformierter Werte, um einen normalen oder abnormalen Zustand des Zeitfensters zu erfassen, wobei der Anomaliedetektor anhand von Sätzen transformierter Werte parametrisiert wird, die aus der Anwendung der mindestens einen Transformation auf Zeitfenster vorhergehender Messungen des mindestens einen Sensors stammen;
- Erfassen einer Anomalie im Wasserkontinuum, entsprechend einer Anzahl von Zeitfenstern, die einen abnormalen Zustand aufweist;
- Ausführen eines Satzes von vordefinierten Regeln zum Erfassen einer Variation von mindestens einer der physikalischen Größen;
- wenn eine Anomalie im Wasserkontinuum erkannt wird, Zuweisen zu der Anomalie eines Typs von Anomalien, der mit einer Variation einer vordefinierten Teilmenge der physikalischen Größen verbunden ist, wenn die Variation mindestens einer der Größen der Teilmenge erkannt wird.

## Claims

1. A device (100, 200) able to detect and characterise anomalies in a water continuum, comprising:
- at least one communication link (221, 222), respectively to at least one sensor (211, 212) for at least one physical quantity in the water continuum;
- a processor (240) configured to:
- receive measurements from the at least one sensor through the at least one communication link;
- generate a plurality of time windows of the measurements;
- for each time window of said plurality:
- obtain a set of values transformed by at least one transformation (242) of the measurements over the time window;
- apply an anomaly detector (243) to the set of transformed values so as to detect a normal or abnormal state of the time window, said anomaly detector being parameterised on the basis of sets of transformed values arising from the application of the at least one transformation to time windows of previous measurements of the at least one sensor;
- detect (244) an anomaly in the water continuum, as a function of a number of time windows exhibiting an abnormal state;
- execute (245) on the measurements a set of predefined rules for detecting a variation of at least one of the physical quantities;
- if an anomaly in the water continuum is detected, assign (246) to the anomaly an anomaly type associated with a variation of a predefined subset of the physical quantities, if the variation of at least one of the quantities of the subset is detected.

2. The device according to claim 1, wherein one of the transformations of the measurements over a time window is a transformation of the measurements into values representative of the variability of the measurements within the time window.

3. The device according to claim 2, wherein the transformation of the measurements into values representative of the variability of the measurements within the time window is carried out by subtracting, from each measurement over the time window, a median of said measurements over a sliding time window comprising at least said time window.

4. The device according to one of claims 1 to 3, wherein the transformation preserves only measurements corresponding to a predefined direction of variation.

5. The device according to claim 1, wherein one of the transformations of the measurements over a time window consists of a slope test over the time window.

6. The device according to claim 1, wherein at least two of the sensors are sensors for one and the same physical quantity at two points of the water continuum, and one of the transformations of the measurements over a time window comprises a time difference between the measurements of the two sensors.

7. The device according to one of claims 1 to 6, wherein the set of transformed values is a vector, and the anomaly detector is a one-class Support Vector Machine.

8. The device according to one of claims 1 to 7, wherein the set of transformed values is obtained by at least two transformations of the measurements over the time window, and the anomaly detector is configured to determine a normal or abnormal state of the time window on the basis of the set of transformed values.

9. The device according to one of claims 1 to 7, wherein the set of transformed values is obtained by at least two transformations of the measurements over the time window, and the anomaly detector is configured to:
- detect normal or abnormal states of at least two subsets of values transformed respectively by said at least two transformations of the measurements over the time window;
- detect the normal or abnormal state of the time window on the basis of a combination of said normal or abnormal states of the subsets.

10. The device according to one of claims 1 to 9, wherein the processor is configured to calculate an intensity of variation of the physical quantities, and the set of predefined characterisation rules comprises a predefined rule for detecting a variation of a physical quantity, if the intensity of variation of said physical quantity over the measurements is greater than a normal variation threshold.

11. The device according to claim 10, wherein the processor is configured to assign a criticality indicator to the anomaly, as a function of the intensities of the variations of the physical quantities of the subset.

12. The device according to one of claims 1 to 11, wherein the detection of the variation of the physical quantity only detects a variation if the variation of the physical quantity complies with a direction of variation.

13. The device according to claims 1 to 12, wherein the processor is configured, if an anomaly is detected, to assign at least one of the following types to the anomaly:
- a "bacterial growth" type, in case of a variation of physical quantities of a subset comprising: a decrease in the chlorine concentration, an increase in temperature, an increase in the total organic carbon content, an increase in the absorbance of ultraviolet light of wavelength 254 nm, an increase in the number of bacteria;
- a "water mixture" type, in case of variations of physical quantities of a subset comprising a conductivity, a pH; a temperature;
- a "coloured waters" type, in case of variations the physical quantities of a subset comprising a chlorine concentration, a pH, an increase in colour, an increase in turbidity, an increase in the absorbance of ultraviolet light of wavelength 254 nm;
- an overspeed, in case of abnormal increase of physical quantities from among a subset comprising turbidity and particles.

14. The device according to one of claims 1 to 13, wherein the processor is configured, if the output of predefined rules for detecting a variation of at least one of the physical quantities does not allow a type to be assigned to an anomaly, to assign an unknown type to this anomaly.

15. The device according to one of claims 1 to 14, comprising an interface (250) for displaying the anomaly and its type to an operator.

16. The device according to claim 15, wherein:
- the interface is configured to receive, from the operator, a label relating to the anomaly;
- the time window of values, and the label relating to the anomaly are added to the training data.

17. A method (800) for detecting and characterising anomalies in a water continuum, comprising:
- the reception (810) of measurements of a plurality of physical quantities arising from a plurality of sensors for the plurality of physical quantities in the water continuum;
- the generation (820) of a plurality of time windows of measurements;
- for each window of said plurality:
- the obtaining of a set of values transformed by at least one transformation (830) of the measurements over the time window;
- the detection (840) of a normal or abnormal state of the time window, said detection being parameterised on the basis of sets of transformed values arising from the application of the at least one transformation to time windows of previous measurements of the at least one sensor;
- the detection (850) of an anomaly in the water continuum, as a function of a number of time windows exhibiting an abnormal state;
- the execution (860) of a set of predefined rules for detecting a variation of at least one of the physical quantities;
- if an anomaly in the water continuum is detected, the assignment (870), to the anomaly, of an anomaly type associated with a variation of a subset of the physical quantities, if the variation of at least one of the quantities of the subset is detected.

18. A computer program product comprising program code instructions stored on a computer-readable medium comprising a processor for the detection of anomalies in a water continuum, said computer program comprising computer-readable programming means for:
- receiving measurements of a plurality of physical quantities arising from a plurality of sensors for the plurality of physical quantities in the water continuum;
- generating a plurality of time windows of measurements;
- for each window of said plurality:
- obtaining a set of values transformed by at least one transformation of the measurements over the time window;
- applying an anomaly detector to the set of transformed values so as to detect a normal or abnormal state of the time window, said anomaly detector being parameterised on the basis of sets of transformed values arising from the application of the at least one transformation to time windows of previous measurements of the at least one sensor;
- detecting an anomaly in the water continuum, as a function of a number of time windows exhibiting an abnormal state;
- executing a set of predefined rules for detecting a variation of at least one of the physical quantities;
- if an anomaly in the water continuum is detected, assigning, to the anomaly, an anomaly type associated with a variation of a predefined subset of the physical quantities, if the variation of at least one of the quantities of the subset is detected.
